# EUROPEAN PATENT APPLICATION

(11) **EP 4 268 795 A1**
(43) Date of publication of application: **01.11.2023**
(21) Application number: 21911109.3
(22) Date of filing: 24.12.2021
(51) Int. Cl.: A61K 6/887, A61C 13/15

(54) **DENTAL COMPOSITION**

(30) Priority: 25.12.2020 JP 2020218032
(71) Applicant: Kuraray Noritake Dental Inc., Kurashiki-shi, Okayama 710-0801 (JP)
(72) Inventor: MATSUURA, Ryo, Tainai-shi, Niigata 959-2653 (JP)
(74) Representative: D Young & Co LLP
(86) International application number: PCT/JP2021/048422
(87) International publication number: WO 2022/138972

(57) **Abstract**

An object of the present invention is to provide a dental composition that exhibits small polymerization shrinkage stress, and provides excellent mechanical strength. The present invention relates to a dental composition comprising a compound (A) having a weight-average molecular weight of 2,000 or more, a monomer (B), and a polymerization initiator (C), wherein the compound (A) having a weight-average molecular weight of 2,000 or more has a glass transition region including a temperature range of 20 to 40°C, and the monomer (B) excludes the compound (A) having a weight-average molecular weight of 2,000 or more. The compound (A) having a weight-average molecular weight of 2,000 or more has a weight-average molecular weight of preferably less than 50,000.

## Description

### TECHNICAL FIELD

The present invention relates to a dental composition used in the field of dental treatment.

### BACKGROUND ART

Restorations using dental bonding materials and dental composite resins have been generally practiced for the treatment of dental caries and associated defects. The restorative treatment typically follows the following procedure. First, the damaged portion including the cavity is removed to create a pit, and a dental bonding material is applied to the pit. The dental bonding material is then cured by irradiating the applied area with visible light. Next, a dental composite resin is filled over the cured dental bonding material layer, and the dental composite resin is cured with visible light.

Dental composite resins have become widely used as alternative materials of conventional metallic materials because of excellent aesthetics comparable to natural teeth, and good ease of handling. A dental composite resin is typically composed mainly of a polymerizable monomer, a polymerization initiator, and a filler. In view of body's safety and the mechanical strength and wear resistance of the cured product, radical polymerizable polyfunctional (meth)acrylates are commonly used as polymerizable monomers. Particularly common are 2,2-bis[4-(3-methacryloyloxy-2-hydroxypropoxy)phenyl]propane (Bis-GMA), and 2,2,4-trimethylhexamethylenebis(2-carbamoyloxyethyl)dimethacrylate (UDMA). Because Bis-GMA and UDMA are highly viscous on its own, these are typically used by being diluted with a polymerizable monomer having a relatively low viscosity, such as triethylene glycol dimethacrylate (3G).

Although dental composite resins are now widely used in clinical practice, there is a need for improvements in the following areas. Specifically, it has been noted that there is a great deal of room for improvement in areas such as ease of handling of a paste of a polymerizable composition used as a dental composite resin, improvement of the flexural strength, elastic modulus, and wear resistance of a cured product, a reduction of water absorption and discoloration or staining, a reduction of polymerization shrinkage stress during cure, and providing aesthetics comparable to natural teeth.

In recent years, there is particularly a strong need to minimize polymerization shrinkage stress because a large polymerization shrinkage stress leads to a contraction gap, which occurs when the dental composite resin pulls away from the bonded surface. Formation of a contraction gap causes various problems, such as secondary caries, tooth pulp stimulation, staining, and detachment of restorations.

Methods that add a polymer to a dental composition have been proposed as a way of reducing such polymerization shrinkage stress. Patent Literature 1 discloses a dental composition that uses a branched polymer to reduce polymerization shrinkage stress. Patent Literature 2 discloses a dental composition that uses a macrocyclic oligomer to reduce polymerization shrinkage stress. Patent Literature 3 discloses a particulate composite material comprised of an organic binder and an inorganic filler. Patent Literature 4 discloses a dental material formed of a composition comprising a metal fine powder, a (meth)acrylic polymer particle, a polymerizable monomer component, and a polymerization catalyst. Patent Literature 5 discloses a dental filling and restorative kit comprising a transparent external filler comprised of particles having a maximum diameter of 0.5 mm to 4.0 mm, and a dental polymerizable composition containing a radical polymerizable monomer and a photopolymerization initiator.

However, Patent Literature 1 cannot provide a sufficient level of reduction in polymerization shrinkage stress, and is also insufficient in terms of strength because the filling rate of inorganic filler cannot be increased high enough due to an increased viscosity of the composition attributed to the branched polymer having a weight-average molecular weight of 20,000 or more. Patent Literature 2 fails to reduce polymerization shrinkage stress to a sufficient level even with the means it discloses. Patent Literature 2 is also insufficient in terms of strength because the macrocyclic oligomer contained increases viscosity, and the filling rate of inorganic filler cannot be increased to a satisfactory level by a phenomenon similar to that observed in Patent Literature 1. In Patent Literature 3, a particulate composite material of large particle size is contained in large amounts, and this produces roughness in the paste. Additionally, because the particulate composite material is treated with a polymerizable functional group, the polymerization shrinkage stress reducing effect was found to be insufficient after polymerization upon cure. Patent Literature 4 is insufficient in terms of mechanical strength as with Patent Literature 3 because the constituent components of the dental material are mostly (meth)acrylic polymer particles. As with Patent Literature 3, Patent Literature 5 is also insufficient in terms of mechanical strength. Additionally, the effect cannot be produced in a reliable fashion because the excessively large particle size leads to some pastes containing particles while others have no particles when a small amount of paste is used in clinical practice.

Other known dental compositions contain an oligomer added to dental compositions (for example, Patent Literatures 6 to 9). However, in Patent Literatures 6 to 9, an oligomer is added with the intention to improve mechanical strength, and the idea of reducing polymerization shrinkage stress is lacking. In addition, studies by the present inventor confirmed that the desired effect of reducing polymerization shrinkage stress was absent.

### CITATION LIST

### Patent Literature

Patent Literature 1: JP 2014-24775 A
Patent Literature 2: JP 2012-188672 A
Patent Literature 3: JP 2002-256010 A
Patent Literature 4: JP H11-29428 A
Patent Literature 5: JP 2016-175851 A
Patent Literature 6: JP S50-042696 A
Patent Literature 7: JP 2006-510583 T
Patent Literature 8: JP 2009-184971 A
Patent Literature 9: JP 2011-144121 A

### SUMMARY OF INVENTION

### Technical Problem

As discussed above, no dental composition is available in related art that exhibits small polymerization shrinkage stress, and provides excellent mechanical strength in the cured product.

It is accordingly an object of the present invention to provide a dental composition that exhibits small polymerization shrinkage stress, and provides excellent mechanical strength in the cured product.

### Solution to Problem

The present inventor conducted intensive studies, and found that the foregoing issues can be solved with a dental composition that comprises a compound having a weight-average molecular weight equal to or above a certain value, and having a glass transition region including a specific temperature range. The present invention was completed after further studies.

Specifically, the present invention includes the following.
[1] A dental composition comprising a compound (A) having a weight-average molecular weight of 2,000 or more, a monomer (B), and a polymerization initiator (C), wherein the compound (A) having a weight-average molecular weight of 2,000 or more has a glass transition region including a temperature range of 20 to 40°C, and the monomer (B) excludes the compound (A) having a weight-average molecular weight of 2,000 or more.
[2] The dental composition according to [1], wherein the compound (A) having a weight-average molecular weight of 2,000 or more has a weight-average molecular weight of less than 50,000.
[3] The dental composition according to [1] or [2], wherein the compound (A) having a weight-average molecular weight of 2,000 or more has a urethane bond.
[4] The dental composition according to [3], wherein the compound (A) having a weight-average molecular weight of 2,000 or more is a urethanized (meth)acrylic compound (A-1).
[5] The dental composition according to any one of [1] to [4], wherein the compound (A) having a weight-average molecular weight of 2,000 or more has a glass transition temperature at 20°C or less.
[6] The dental composition according to any one of [1] to [5], wherein the compound (A) having a weight-average molecular weight of 2,000 or more is 0.1 to 50 parts by mass in total 100 parts by mass of the compound (A) having a weight-average molecular weight of 2,000 or more, and the monomer (B).
[7] The dental composition according to any one of [1] to [6], wherein the compound (A) having a weight-average molecular weight of 2,000 or more has two or more glass transition temperatures.
[8] The dental composition according to [7], wherein the compound (A) having a weight-average molecular weight of 2,000 or more has one or more glass transition temperatures in a temperature region of -100°C or more and 20°C or less, and one or more glass transition temperatures in a temperature region of 20°C or more and less than 80°C.
[9] The dental composition according to any one of [1] to [8], wherein the monomer (B) comprises a monomer (B-1) having an acidic group.
[10] The dental composition according to any one of [1] to [9], which further comprises a filler (D).
[11] The dental composition according to any one of [1] to [10], wherein the compound (A) having a weight-average molecular weight of 2,000 or more has a polymerizable group, and has a weight-average molecular weight of 1,250 or more and less than 20,000 per its polymerizable group.
[12] A dental composite resin comprising a dental composition of any one of [1] to [11].
[13] A self-adhesive dental composite resin comprising a dental composition of any one of [1] to [11].
[14] A dental cement comprising a dental composition of any one of [1] to [11].

### Advantageous Effects of Invention

According to the present invention, a dental composition can be provided that exhibits small polymerization shrinkage stress, and provides excellent mechanical strength in the cured product. The dental composition can be suitably used as, for example, a dental composite resin, a self-adhesive dental composite resin, or a dental cement.

### BRIEF DESCRIPTION OF DRAWINGS

FIG. 1 is a schematic view illustrating how a glass transition temperature is determined when glass transition shows a step-like change as in FIG. 3 of JIS K 7121-1987 representing a method of determination of glass transition temperature.
FIG. 2 shows a result of DSC measurement for UN-7600 used in Example.

### DESCRIPTION OF EMBODIMENTS

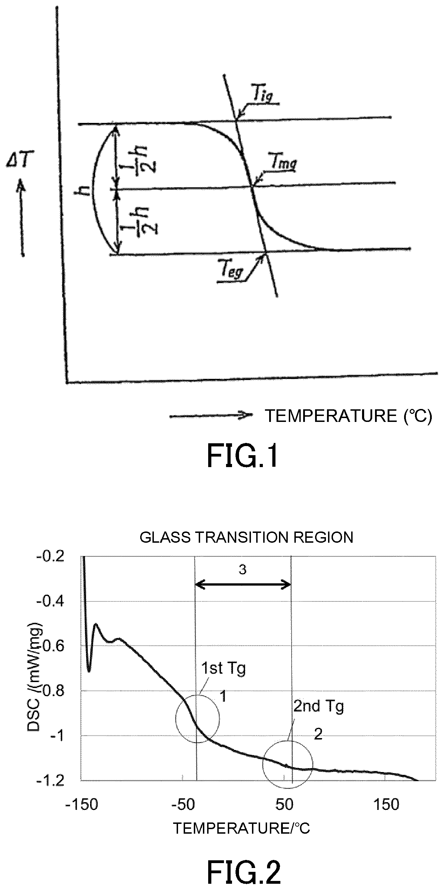

A dental composition of the present invention is a dental composition comprising a compound (A) having a weight-average molecular weight of 2,000 or more, a monomer (B), and a polymerization initiator (C), wherein the compound (A) having a weight-average molecular weight of 2,000 or more has a glass transition region including a whole temperature range of 20 to 40°C, and the monomer (B) excludes the compound (A) having a weight-average molecular weight of 2,000 or more. A glass transition region in the present invention is defined as the width of the temperature region from the first inflection point on the generated endothermic peak (the first inflection point on the low-temperature side) to the point where the endothermic peak ends (the last inflection point on the high-temperature side) in heat flux differential scanning calorimetry (DSC). Specifically, a glass transition region in the present invention can be determined by measurement based on JIS K 7121-1987 (rev. 2012). In the following, "baseline" means a DTA or DSC curve in a temperature region in which glass transitions or reactions do not occur in the measurement sample, as defined in JIS K 7121-1987 (rev. 2012). The glass transition region means a temperature range from an extrapolated glass transition onset temperature (T_{ig}) to an extrapolated glass transition end temperature (T_{eg}). The extrapolated glass transition onset temperature (T_{ig}) is the temperature where a straight line as an extension of the baseline on the low-temperature side toward the high-temperature side intersects the tangent to the steepest slope of the curve where the glass transition shows a step-like change. The extrapolated glass transition end temperature (T_{eg}) is the temperature where the straight line as an extension of the baseline on the high-temperature side toward the low-temperature side intersects the tangent to the steepest slope of the curve where the glass transition shows a step-like change. FIG. 1 shows the extrapolated glass transition onset temperature (T_{ig}), extrapolated glass transition end temperature (T_{eg}), and midpoint glass transition temperature (T_{mg}). When the compound (A) having a weight-average molecular weight of 2,000 or more is a compound with two or more glass transition temperatures, the glass transition region means the temperature range from the first T_{ig} on the low-temperature side to the last T_{eg} on the high-temperature side. In heat flux DSC, the extrapolated glass transition end temperature (T_{eg}) of when a peak appears on the high-temperature side of a step-like change is the temperature where the straight line as an extension of the baseline on the high-temperature side toward the low-temperature side intersects the tangent to the steepest slope of the curve on the high-temperature side of the peak. In the present invention, a glass transition temperature (Tg) means the midpoint glass transition temperature (T_{mg}). The midpoint glass transition temperature (T_{mg}) is the temperature where the straight line vertically equidistant from the straight line as an extension of each baseline intersects the curve where the glass transition shows a step-like change.

The measurement methods and measurement conditions related to the glass transition temperature and glass transition region of the present invention are as described in the EXAMPLES section below using heat flux DSC.

In the present specification, "(meth)acryl" is a collective term for methacryl and acryl, and the same applies to similar expressions, such as "(meth)acrylic acid" and "(meth)acrylonitrile". In the present specification, the upper limits and lower limits of numeric ranges (for example, ranges of contents of components, ranges of values calculated from components, and ranges of physical properties) can be appropriately combined.

The reason a dental composition of the present invention exhibits small polymerization shrinkage stress during polymerization and cure is unclear. However, the following speculation has been made. Given that a dental composition contains a compound (A) having a weight-average molecular weight of 2,000 or more, and the glass transition region of the compound (A) includes the whole range from 20 to 40°C, it can be speculated that the shrinkage stress that generates in the dental composition when it cures attenuates as the kinetic energy of shrinkage is converted into heat energy, as detailed below. In polymerization and curing of a dental composition, curing proceeds from the area exposed to light in the case of photo-polymerization, and from the center in the case of chemical polymerization. Here, shrinkage stress acts toward the area where the curing has started, and causes the bonding interface to experience stress, leading to a decrease of adhesive properties or formation of a contraction gap. In the case of a dental composition of the present invention comprising a compound (A) having a weight-average molecular weight of 2,000 or more, the glass transition region of the compound (A) having a weight-average molecular weight of 2,000 or more includes the polymerization temperature (room temperature to the temperature inside the oral cavity; 20°C to 40°C). Accordingly, the main chain of the molecule undergoes active micro-Brownian movement, and the loss tangent (Tan δ) is high. When the dental composition experiences polymerization shrinkage stress in such a glass transition region, the stress causes a change in the state of the molecular chain segment while the molecular chain segment attempts to return to its original state at the same time. This creates friction between molecules or within the molecule, converting the kinetic energy into heat energy, and reducing the polymerization shrinkage stress as a result.

The following describes the components used in a dental composition of the present invention.

### [Compound (A) Having Weight-Average Molecular Weight of 2,000 or More]

The compound (A) having a weight-average molecular weight of 2,000 or more (hereinafter, also referred to as "compound (A)") has a glass transition region including the whole temperature range from 20 to 40°C. In a dental composition of the present invention, the compound (A) is used to provide a polymerization shrinkage stress reducing effect. The compound (A) may be used alone, or two or more thereof may be used in combination. A certain preferred embodiment may be, for example, a dental composition in which the compound (A) is a compound having a urethane bond.

In view of the mechanical strength of a cured product of the dental composition, the compound (A) preferably comprises a compound having a polymerizable group. Examples of the compound (A) include compounds having a polymerizable group, and additionally, a urethane bond. Examples of the polymerizable group include a vinyl group, a (meth)acryl group, and a (meth)acrylamide group. The polymerizable group is preferably a (meth)acryl group or a (meth)acrylamide group, more preferably a (meth)acryl group. A compound having a weight-average molecular weight of 2,000 or more with no polymerizable group will be described later. The following describes a compound (A) that has a (meth)acryl group as a polymerizable group. A compound (A) having a (meth)acryl group can be broadly classified into a urethanized (meth)acrylic compound (A-1) having a urethane bond (hereinafter, also referred to as "urethanized (meth)acrylic compound (A-1)"), and a (meth)acrylic compound (A-2) having no urethane bond. The urethanized (meth)acrylic compound (A-1) is preferred in view of ease of introducing a (meth)acryl group, and the polymerization shrinkage stress reducing effect.

### · Urethanized (Meth)Acrylic Compound (A-1)

The urethanized (meth)acrylic compound (A-1) can be easily synthesized by, for example, an addition reaction of a polyol containing the polymer skeleton (described later), a compound having an isocyanate group (-NCO), and a (meth)acrylic compound having a hydroxyl group (-OH). Alternatively, the urethanized (meth)acrylic compound (A-1) can be synthesized with ease by, for example, allowing lactone or alkylene oxide to undergo a ring-opening addition reaction with a (meth)acrylic compound having a hydroxyl group, and causing the resulting compound having a terminal hydroxyl group to undergo an addition reaction with a compound having an isocyanate group. The (meth)acrylic compound that donates a (meth)acryl group to a polymer can introduce a (meth)acryl group to, for example, a polymer of a monomer having a hydroxyl group through a dehydrocondensation reaction of (meth)acrylic acid.

The urethanized (meth)acrylic compound (A-1) is preferably a (meth)acrylate having a structure (a polymer skeleton) selected from the group consisting of a polyester, a polycarbonate, a polyurethane, a polyether, a poly-conjugated diene, and a hydrogenated poly-conjugated diene, in addition to a urethane bond. More preferably, the urethanized (meth)acrylic compound (A-1) is a (meth)acrylate having, within the molecule, a urethane bond, and at least one polyol moiety selected from the group consisting of a polyester, a polycarbonate, a polyurethane, a polyether, a poly-conjugated diene, and a hydrogenated poly-conjugated diene each having a structure derived from a C4 to C18 aliphatic diol unit having a branched structure. These are not particularly limited, as long as the above structure is present.

Examples of the polyester include: a polymer of a dicarboxylic acid (e.g., an aromatic dicarboxylic acid such as phthalic acid or isophthalic acid, or an unsaturated aliphatic dicarboxylic acid such as maleic acid) and an aliphatic diol having 2 to 18 carbon atoms; a polymer of a dicarboxylic acid (e.g., a saturated aliphatic dicarboxylic acid such as adipic acid or sebacic acid) and an aliphatic diol having 2 to 18 carbon atoms; a polymer of β-propiolactone; a polymer of γ-butyrolactone; a polymer of δ-valerolactone; a polymer of ε-caprolactone; and a copolymer of these. Preferred are a polymer of a dicarboxylic acid (an aromatic dicarboxylic acid such as phthalic acid or isophthalic acid, or an unsaturated aliphatic dicarboxylic acid such as maleic acid) and an aliphatic diol having 2 to 12 carbon atoms; and a polymer of a dicarboxylic acid (a saturated aliphatic dicarboxylic acid such as adipic acid or sebacic acid) and an aliphatic diol having 2 to 12 carbon atoms.

Examples of the polycarbonate include a polycarbonate derived from an aliphatic diol having 2 to 18 carbon atoms, a polycarbonate derived from bisphenol A, and a polycarbonate derived from a C2 to C18 aliphatic diol and bisphenol A. Preferred are a polycarbonate derived from an aliphatic diol having 2 to 12 carbon atoms, a polycarbonate derived from bisphenol A, and a polycarbonate derived from a C2 to C12 aliphatic diol and bisphenol A.

Examples of the polyurethane include a polymer of a C2 to C18 aliphatic diol and a C1 to C18 diisocyanate. Preferred is a polymer of a C2 to C12 aliphatic diol and a C1 to C12 diisocyanate.

Examples of the polyether include polyethylene glycol, polypropylene glycol, polybutylene glycol, and poly(1-methylbutylene glycol).

Examples of the poly-conjugated diene and hydrogenated poly-conjugated diene include 1,4-polybutadiene, 1,2-polybutadiene, polyisoprene, poly(butadiene-isoprene), poly(butadiene-styrene), poly(isoprene-styrene), polyfarnesene, and hydrogenated products of these.

Among these structures, a polyester, a polycarbonate, and a poly-conjugated diene are preferred in view of superior flexibility and superior water resistance. A polyol having the polymer skeleton mentioned above can be used for the production of urethanized (meth)acrylic compound (A-1). By adjusting the skeleton or molecular weight of a structure (a polymer skeleton) selected from the group consisting of a polyester, a polycarbonate, a polyurethane, a polyether, a poly-conjugated diene, and a hydrogenated poly-conjugated diene, it is easier to set the glass transition region within the desired range, and to adjust a compound having two or more glass transition temperatures in a specific temperature region.

Examples of the compound having an isocyanate group include hexamethylene diisocyanate (HDI), tolylene diisocyanate (TDI), xylylene diisocyanate (XDI), diphenylmethane diisocyanate (MDI), isophorone diisocyanate (IPDI), trimethylhexamethylene diisocyanate (TMHMDI), tricyclodecane diisocyanate (TCDDI), and adamantane diisocyanate (ADI).

Examples of the (meth)acrylic compound having a hydroxyl group include:
hydroxy (meth)acrylate compounds such as 2-hydroxyethyl (meth)acrylate, 2-hydroxypropyl (meth)acrylate, 2-hydroxybutyl (meth)acrylate, 6-hydroxyhexyl (meth)acrylate, 10-hydroxydecyl (meth)acrylate, 3-chloro-2-hydroxypropyl (meth)acrylate, 2-hydroxy-3-phenoxypropyl (meth)acrylate, glycerin mono(meth)acrylate, 2-hydroxy-3-acryloyloxypropyl (meth)acrylate, 2,2-bis[4-[3-(meth)acryloyloxy-2-hydroxypropoxy]phenyl]propane, 1,2-bis[3-(meth)acryloyloxy-2-hydroxypropoxy]ethane, pentaerythritol tri(meth)acrylate, and tri or tetra(meth)acrylates of dipentaerythritol; and
hydroxy (meth)acrylamide compounds such as N-hydroxyethyl (meth)acrylamide, and N,N-bis(2-hydroxyethyl)(meth)acrylamide.

Examples of the C4 to C18 aliphatic diol having a branched structure include 2-methyl-1,3-propanediol, 2,2-diethyl-1,3-propanediol, 1,3-butanediol, 2-methyl-1,4-butanediol, neopentyl glycol, 3-methyl-1,5-pentanediol, 2-methyl-1,8-octanediol, 2,7-dimethyl-1,8-octanediol, 2-methyl-1,9-nonanediol, 2,8-dimethyl-1,9-nonanediol, 2-methyl-1,10-decanediol, 2,9-dimethyl-1,10-decanediol, 2-methyl-1,11-undecanediol, 2,10-dimethyl-1,11-undecanediol, 2-methyl-1,12-dodecanediol, 2,11-dimethyl-1,12-dodecanediol, 2-methyl-1,13-tridecanediol, 2,12-dimethyl-1,13-tridecanediol, 2-methyl-1,14-tetradecanediol, 2,13-dimethyl-1,14-tetradecanediol, 2-methyl-1,15-pentadecanediol, 2,14-dimethyl-1,15-pentadecanediol, 2-methyl-1,16-hexadecanediol, and 2,15-dimethyl-1,16-hexadecanediol. In view of providing a dental composition having superior curability, the polyol components used are preferably C5 to C12 aliphatic diols having a methyl-group side chain, for example, such as 2-methyl-1,4-butanediol, 3-methyl-1,5-pentanediol, 2-methyl-1,8-octanediol, 2,7-dimethyl-1,8-octanediol, 2-methyl-1,9-nonanediol, and 2,8-dimethyl-1,9-nonanediol. The polyol components are more preferably 2-methyl-1,4-butanediol, 3-methyl-1,5-pentanediol, 2-methyl-1,8-octanediol, and 2,7-dimethyl-1,8-octanediol, even more preferably 3-methyl-1,5-pentanediol, and 2-methyl-1,8-octanediol.

The addition reaction of the compound having an isocyanate group and the (meth)acrylic compound having a hydroxyl group can be performed following a known method, and the method is not particularly limited. It is easier to set the glass transition region within the desired range, and to adjust a compound having two or more glass transition temperatures in a specific temperature region when a catalyst is used in the production method. The catalyst is not particularly limited, and may be, for example, a dibutyltin compound such as di-n-butyltin dilaurate, dibutyltin dichloride, dibutyltin malate, dibutyltin oxide, and dibutyl diacetate.

The urethanized (meth)acrylic compound (A-1) obtained is, for example, a product of a reaction of any combination of: the polyol having at least one structure selected from the group consisting of a polyester, a polycarbonate, a polyurethane, a polyether, a poly-conjugated diene, and a hydrogenated poly-conjugated diene; the compound having an isocyanate group; and the (meth)acrylic compound having a hydroxyl group. In certain embodiments, the urethanized (meth)acrylic compound (A-1) is preferably a urethanized (meth)acrylic compound (A-1a) that does not comprise an aromatic ring in the skeleton, in order to prevent an increase of glass transition temperature, and adjust the glass transition region within 20 to 40°C. In other embodiments, the urethanized (meth)acrylic compound (A-1) is preferably a urethanized (meth)acrylic compound (A-1b) that does not comprise a cyclic structure (an aromatic ring, a heterocyclic ring, or an alicyclic structure) in the skeleton, in order to adjust the glass transition region below 40°C.

Yet another embodiment is, for example, a dental composition that comprises a compound (A) having a weight-average molecular weight of 2,000 or more, a monomer (B), and a polymerization initiator (C), wherein the compound (A) having a weight-average molecular weight of 2,000 or more has a glass transition region including a temperature range of 20 to 40°C, and the monomer (B) is a compound that excludes the compound (A) having a weight-average molecular weight of 2,000 or more,
the dental composition comprising no compound that has a weight-average molecular weight of 2,000 or more but does not have a glass transition region including a temperature range of 20 to 40°C (for example, such as a (meth)acrylic compound).

### · (Meth)Acrylic Compound (A-2) Having no Urethane Bond

The (meth)acrylic compound (A-2) having no urethane bond has a structure (a polymer skeleton) selected from the group consisting of a polyester, a polycarbonate, a polyurethane, a polyether, a poly-conjugated diene, and a hydrogenated poly-conjugated diene. These are not particularly limited, as long as the above structure is present.

Examples of the polyester include a polymer of phthalic acid and an alkylene diol having 2 to 12 carbon atoms, a polymer of adipic acid and an alkylene glycol having 2 to 12 carbon atoms, a polymer of maleic acid and an alkylene diol having 2 to 12 carbon atoms, a polymer of β-propiolactone, a polymer of γ-butyrolactone, a polymer of δ-valerolactone, a polymer of ε-caprolactone, and a copolymer of these.

Examples of the polycarbonate include a polycarbonate derived from an aliphatic diol having 2 to 12 carbon atoms, a polycarbonate derived from bisphenol A, and a polycarbonate derived from a C2 to C12 aliphatic diol and bisphenol A.

Examples of the polyurethane include a polymer of a C2 to C12 aliphatic diol and a C1 to C12 diisocyanate.

Examples of the polyether include polyethylene glycol, polypropylene glycol, polybutylene glycol, and poly(1-methylbutylene glycol).

Examples of the poly-conjugated diene and hydrogenated poly-conjugated diene include 1,4-polybutadiene, 1,2-polybutadiene, polyisoprene, poly(butadiene-isoprene), poly(butadiene-styrene), poly(isoprene-styrene), polyfarnesene, and hydrogenated products of these.

In view of superior flexibility and superior water resistance, preferred among these are the polyester, polycarbonate, and poly-conjugated diene structures. A polyol having the polymer skeleton mentioned above can be used for the production of (meth)acrylic compound (A-2) having no urethane bond.

Examples of the (meth)acrylic compound having a hydroxyl group include:
hydroxy (meth)acrylate compounds such as 2-hydroxyethyl (meth)acrylate, 2-hydroxypropyl (meth)acrylate, 2-hydroxybutyl (meth)acrylate, 6-hydroxyhexyl (meth)acrylate, 10-hydroxydecyl (meth)acrylate, 3-chloro-2-hydroxypropyl (meth)acrylate, 2-hydroxy-3-phenoxypropyl (meth)acrylate, glycerin mono(meth)acrylate, 2-hydroxy-3-acryloyloxypropyl (meth)acrylate, 2,2-bis[4-[3-(meth)acryloyloxy-2-hydroxypropoxy]phenyl]propane, 1,2-bis[3-(meth)acryloyloxy-2-hydroxypropoxy]ethane, pentaerythritol tri(meth)acrylate, and tri or tetra(meth)acrylates of dipentaerythritol; and
hydroxy (meth)acrylamide compounds such as N-hydroxyethyl (meth)acrylamide, and N,N-bis(2-hydroxyethyl)(meth)acrylamide.

The (meth)acrylic compound (A-2) having no urethane bond obtained is, for example, a product of a reaction of any combination of: the polyol having at least one structure selected from the group consisting of a polyester, a polycarbonate, a polyurethane, a polyether, a poly-conjugated diene, and a hydrogenated poly-conjugated diene; and the (meth)acrylic compound having a hydroxyl group.

### · Compound Having Weight-Average Molecular Weight of 2,000 or More with no Polymerizable Group

The compound having a weight-average molecular weight of 2,000 or more with no polymerizable group can be classified into a compound (A-3) having a weight-average molecular weight of 2,000 or more with a urethane bond and with no polymerizable group (hereinafter, also referred to as "compound (A-3)"), and a compound (A-4) having a weight-average molecular weight of 2,000 or more with no urethane bond or polymerizable group (hereinafter, also referred to as "compound (A-4)"). Preferred is compound (A-3). A certain embodiment is, for example, a dental composition that comprises a compound (A), a monomer (B), and a polymerization initiator (C), but does not comprise a compound (A-3) and/or a compound (A-4).

The compound (A-3) has a structure (a polymer skeleton) selected from the group consisting of a polyester, a polycarbonate, a polyurethane, a polyether, a poly-conjugated diene, and a hydrogenated poly-conjugated diene, in addition to a urethane bond. These are not particularly limited, as long as the above structure is present.

Examples of the polyester include a polymer of phthalic acid and an alkylene diol having 2 to 12 carbon atoms, a polymer of adipic acid and an alkylene glycol having 2 to 12 carbon atoms, a polymer of maleic acid and an alkylene diol having 2 to 12 carbon atoms, a polymer of β-propiolactone, a polymer of γ-butyrolactone, a polymer of δ-valerolactone, a polymer of ε-caprolactone, and a copolymer of these.

Examples of the polycarbonate include a polycarbonate derived from an aliphatic diol having 2 to 12 carbon atoms, a polycarbonate derived from bisphenol A, and a polycarbonate derived from a C2 to C12 aliphatic diol and bisphenol A.

Examples of the polyurethane include a polymer of a C2 to C12 aliphatic diol and a C1 to C12 diisocyanate.

Examples of the polyether include polyethylene glycol, polypropylene glycol, polybutylene glycol, and poly(1-methylbutylene glycol).

Examples of the poly-conjugated diene and hydrogenated poly-conjugated diene include 1,4-polybutadiene, 1,2-polybutadiene, polyisoprene, poly(butadiene-isoprene), poly(butadiene-styrene), poly(isoprene-styrene), polyfarnesene, and hydrogenated products of these.

In view of superior flexibility and superior water resistance, preferred among these are the polyester, polycarbonate, and poly-conjugated diene structures. A polyol having the polymer skeleton mentioned above can be used for the production of the compound (A-3) having a weight-average molecular weight of 2,000 or more with no polymerizable group.

Examples of the compound having an isocyanate group include hexamethylene diisocyanate (HDI), tolylene diisocyanate (TDI), xylylene diisocyanate (XDI), diphenylmethane diisocyanate (MDI), isophorone diisocyanate (IPDI), trimethylhexamethylene diisocyanate (TMHMDI), tricyclodecane diisocyanate (TCDDI), and adamantane diisocyanate (ADI).

The compound having a hydroxyl group is not particularly limited, and known compounds can be used, as long as it does not have a polymerizable group. The compound having a hydroxyl group may be, for example, the polyol described above.

The addition reaction of the compound having a isocyanate group and the compound having a hydroxyl group can be carried out following a known method, and is not particularly limited.

The compound (A-3) obtained may be, for example, a product of a reaction between the polyol having at least one structure selected from the group consisting of a polyester, a polycarbonate, a polyurethane, a polyether, a poly-conjugated diene, and a hydrogenated poly-conjugated diene; and the compound having an isocyanate group.

In view of the polymerization shrinkage stress reducing effect, the compound (A) having a weight-average molecular weight of 2,000 or more is preferably hydrophobic. In view of the polymerization shrinkage stress reducing effect, the compound (A) having a weight-average molecular weight of 2,000 or more is preferably not compatible with the other monomer components.

Because the weight-average molecular weight (Mw) of compound (A) is a contributing factor for providing the desired glass transition region and the desired glass transition temperature, and, in view of viscosity and the polymerization shrinkage stress reducing effect, the compound (A) has a weight-average molecular weight (Mw) of 2,000 or more, preferably 3,000 to 50,000, more preferably 5,000 to 20,000. In the present invention, a weight-average molecular weight (Mw) means a weight-average molecular weight determined by gel permeation chromatography (GPC) in terms of polystyrene, and can be measured by using a known method.

The glass transition region of compound (A) must include the whole temperature range from 20 to 40°C, in order to produce the polymerization shrinkage stress reducing effect. The dental composition may experience a temperature rise during polymerization; however, the polymerization shrinkage stress reducing effect should be obtained also during polymerization and cure when the glass transition region includes the temperature of polymerization. In view of these, it is preferable that the glass transition region have the widest temperature range possible. Preferably, the glass transition region extends over a temperature range of 25°C or higher (for example, the glass transition region includes 20 to 45°C), more preferably 30°C or higher (for example, the glass transition region includes 20 to 50°C), even more preferably 35°C or higher (for example, the glass transition region includes 20 to 55°C), particularly preferably 40°C or higher (for example, the glass transition region includes 20 to 60°C). In certain embodiments, the glass transition region may be 25°C or higher.

The compound (A) having a weight-average molecular weight of 2,000 or more (preferably, urethanized (meth)acrylic compound (A-1)) has a weight-average molecular weight of preferably 1,250 or more and less than 20,000, more preferably 1,500 or more and 17,500 or less, even more preferably 1,800 or more and 16,000 or less, particularly preferably 2,500 or more and 15,000 or less per polymerizable group. With the number of polymerizable groups confined in these ranges in the urethanized (meth)acrylic compound (A-1), crosslinkage takes place in a moderate fashion, and it is possible to more effectively maintain the mechanical strength while reducing polymerization shrinkage stress.

In view of the polymerization shrinkage stress reducing effect, the compound (A) having a weight-average molecular weight of 2,000 or more preferably has a glass transition temperature (hereinafter, also referred to simply as "Tg") at 20°C or less. The compound (A) has a Tg of preferably -100°C to 20°C, more preferably -75°C to 15°C, even more preferably -60°C to 10°C.

In certain embodiments, the compound (A) preferably has two or more glass transition temperatures, more preferably two glass transition temperatures. In two or more glass transition temperatures Tg, it is preferable that one or more glass transition temperatures Tg be present on the low-temperature side and on the high-temperature side. In such embodiments, a first glass transition temperature Tg (on the low-temperature side) is preferably -100°C or more and 20°C or less, more preferably -75°C or more and 15°C or less, even more preferably -60°C or more and 10°C or less. In such embodiments, a second glass transition temperature Tg (on the high-temperature side) is preferably 20°C or more and less than 80°C, more preferably 25°C or more and less than 70°C, even more preferably 30°C or more and less than 65°C. A certain embodiment is, for example, a dental composition in which the compound (A) having a weight-average molecular weight of 2,000 or more has one or more glass transition temperatures in the temperature region of -100°C or more and 20°C or less, and one or more glass transition temperatures in the temperature region of 20°C or more and less than 80°C.

Another certain embodiment is, for example, a dental composition that comprises a compound (A) having a weight-average molecular weight of 2,000 or more, a monomer (B), and a polymerization initiator (C), wherein the compound (A) having a weight-average molecular weight of 2,000 or more has a glass transition region including a temperature range of 20 to 40°C, and the monomer (B) is a compound that excludes the compound (A) having a weight-average molecular weight of 2,000 or more,
the dental composition comprising no compound that has a weight-average molecular weight of 2,000 or more but does not have two or more glass transition temperatures (for example, such as a (meth)acrylic compound).

Yet another embodiment is, for example, a dental composition that comprises a compound (A) having a weight-average molecular weight of 2,000 or more, a monomer (B), and a polymerization initiator (C), wherein the compound (A) having a weight-average molecular weight of 2,000 or more has a glass transition region including a temperature range of 20 to 40°C, and the monomer (B) is a compound that excludes the compound (A) having a weight-average molecular weight of 2,000 or more,
the dental composition comprising no compound that has a weight-average molecular weight of 2,000 or more but does not have a glass transition region including a temperature range of 20 to 40°C, and does not have two or more glass transition temperatures (for example, such as a (meth)acrylic compound).

In view of ease of handling and the polymerization shrinkage stress reducing effect, the compound (A) having a weight-average molecular weight of 2,000 or more has a viscosity at 25°C of preferably 5,000 to 10,000,000 cps, more preferably 10,000 to 7,500,000 cps, even more preferably 20,000 to 7,000,000 cps. In the present invention, a viscosity means a viscosity measured at 25°C with a Brookfield viscometer. Measurement conditions such as time and rotational speed are appropriately adjusted according to the viscosity range.

The compound (A) having a weight-average molecular weight of 2,000 or more may be a commercially available product. Examples of such commercially available products include the Art Resin series (UN-7600; viscosity: 1,100,000 cps/25°C, weight-average molecular weight (Mw): 11,500) manufactured by Negami Chemical Industrial Co., Ltd., the Kuraprene series (UC-102M, UC-203M; polyisoprene skeleton or polybutadiene skeleton) manufactured by Kuraray Co., Ltd., and the liquid polybutadiene NISSO-PB manufactured by Nippon Soda Co., Ltd.

In view of the mechanical strength, paste properties, and polymerization shrinkage stress reducing effect, the content of the compound (A) having a weight-average molecular weight of 2,000 or more in a dental composition of the present invention is preferably 0.1 to 50 parts by mass, more preferably 0.5 to 40 parts by mass, even more preferably 1 to 35 parts by mass, yet more preferably 1 to 25 parts by mass, particularly preferably 1 to 18 parts by mass with respect to total 100 parts by mass of the compound (A) having a weight-average molecular weight of 2,000 or more, and the monomer (B). In certain embodiments, in view of the mechanical strength, paste properties, and polymerization shrinkage stress reducing effect, the content of compound (A) having a weight-average molecular weight of 2,000 or more is preferably 0.1 mass% or more, more preferably 0.5 mass% or more, even more preferably 1 mass% or more in a total amount of the dental composition. In view of the mechanical strength, paste properties, and polymerization shrinkage stress reducing effect, the content of compound (A) having a weight-average molecular weight of 2,000 or more is preferably 30 mass% or less, more preferably 25 mass% or less, even more preferably 20 mass% or less in a total amount of the dental composition.

### [Monomer (B)]

Examples of the monomer (B) include a monomer (B-1) having an acidic group, a hydrophobic monomer (B-2) having no acidic group, and a hydrophilic monomer (B-3) having no acidic group. The monomer (B) may be used alone, or two or more thereof may be used in combination. The monomer (B) excludes the compound (A) having a weight-average molecular weight of 2,000 or more. When a certain compound belongs to both a compound (A) having a weight-average molecular weight of 2,000 or more, and a monomer (B), it is regarded as a compound (A) having a weight-average molecular weight of 2,000 or more. When the monomer (B) includes a polymer skeleton, the monomer (B) has a weight-average molecular weight of preferably less than 2,000. When the monomer (B) does not include a polymer skeleton, the monomer (B) has a molecular weight, instead of a weight-average molecular weight, of less than 2,000, because the concept of weight-average molecular weight does not apply to such monomers.

### · Monomer (B-1) Having Acidic Group

The monomer (B-1) having an acidic group has an acid etching effect and a priming effect, and is a component that provides demineralizing effect and penetrative effect. The monomer (B-1) having an acidic group is polymerizable, and provides curing effect. The adhesive properties to tooth structure, and bond durability improve by containing the monomer (B-1) having an acidic group.

The monomer (B-1) having an acidic group is, for example, a monomer having at least one acidic group (such as a phosphoric acid group, a pyrophosphoric acid group, a thiophosphoric acid group, a phosphonic acid group, a sulfonic acid group, and a carboxylic acid group), and at least one polymerizable group (such as a (meth)acryloyl group, a vinyl group, and a styrene group). In view of adhesive properties to tooth structure, the monomer (B-1) having an acidic group is preferably a phosphoric acid group-containing monomer. Specific examples of the monomer (B-1) having an acidic group are as follows.

Examples of the phosphoric acid group-containing monomer include 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, bis[2-(meth)acryloyloxyethyl]hydrogen phosphate, bis[4-(meth)acryloyloxybutyl]hydrogen phosphate, bis[6-(meth)acryloyloxyhexyl]hydrogen phosphate, bis[8-(meth)acryloyloxyoctyl]hydrogen phosphate, bis[9-(meth)acryloyloxynonyl]hydrogen phosphate, bis[10-(meth)acryloyloxydecyl]hydrogen phosphate, 1,3- di(meth)acryloyloxypropyl dihydrogen phosphate, 2-(meth)acryloyloxyethylphenyl hydrogen phosphate, 2-(meth)acryloyloxyethyl-2-bromoethyl hydrogen phosphate, bis[2-(meth)acryloyloxy-(1-hydroxymethyl)ethyl]hydrogen phosphate, and acid chlorides, alkali metal salts, and ammonium salts of these.

Examples of the pyrophosphoric acid group-containing monomer include bis[2-(meth)acryloyloxyethyl]pyrophosphate, bis[4-(meth)acryloyloxybutyl]pyrophosphate, bis[6-(meth)acryloyloxyhexyl]pyrophosphate, bis[8-(meth)acryloyloxyoctyl]pyrophosphate, bis[10-(meth)acryloyloxydecyl]pyrophosphate, and acid chlorides, alkali metal salts, and ammonium salts of these.

Examples of the thiophosphoric acid group-containing monomer include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 16-(meth)acryloyloxyhexadecyl dihydrogen thiophosphate, 20-(meth)acryloyloxyicosyl dihydrogen thiophosphate, and acid chlorides, alkali metal salts, and ammonium salts of these.

Examples of the phosphonic acid group-containing monomer include 2-(meth)acryloyloxyethylphenyl phosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexyl-3-phosphonoacetate, 10-(meth)acryloyloxydecyl-3-phosphonoacetate, and acid chlorides, alkali metal salts, and ammonium salts of these.

Examples of the sulfonic acid group-containing monomer include 2-(meth)acrylamide-2-methylpropanesulfonic acid, styrenesulfonic acid, and 2-sulfoethyl (meth)acrylate.

Examples of the carboxylic acid group-containing monomer include monomers having one carboxy group within the molecule, and monomers having a plurality of carboxy groups within the molecule.

Examples of monomers having one carboxy group within the molecule include (meth)acrylic acid, N-(meth)acryloylglycine, N-(meth)acryloylaspartic acid, O-(meth)acryloyltyrosine, N-(meth)acryloyltyrosine, N-(meth)acryloylphenylalanine, N-(meth)acryloyl-p-aminobenzoic acid, N-(meth)acryloyl-o-aminobenzoic acid, p-vinyl benzoic acid, 2-(meth)acryloyloxybenzoic acid, 3-(meth)acryloyloxybenzoic acid, 4-(meth)acryloyloxybenzoic acid, N-(meth)acryloyl-5-aminosalicylic acid, N-(meth)acryloyl-4-aminosalicylic acid, 2-(meth)acryloyloxyethyl hydrogen succinate, 2-(meth)acryloyloxyethyl hydrogen phthalate, 2-(meth)acryloyloxyethyl hydrogen malate, and acid halides of these.

Examples of monomers having a plurality of carboxy groups within the molecule include 6-(meth)acryloyloxyhexane-1,1-dicarboxylic acid, 9- (meth)acryloyloxynonane-1,1 -dicarboxylic acid, 10-(meth)acryloyloxydecane-1,1-dicarboxylic acid, 11-(meth)acryloyloxyundecane-1,1-dicarboxylic acid, 12-(meth)acryloyloxydodecane-1,1-dicarboxylic acid, 13-(meth)acryloxytridecane-1,1-dicarboxylic acid, 4-(meth)acryloyloxyethyltrimellitate, 4-(meth)acryloyloxyethyltrimellitate anhydride, 4-(meth)acryloyloxybutyltrimellitate, 4-(meth)acryloyloxyhexyltrimellitate, 4-(meth)acryloyloxydecyltrimellitate, 2-(meth)acryloyloxyethyl-3'-(meth)acryloyloxy-2'-(3,4-dicarboxybenzoyloxy)propyl succinate, and acid anhydrides or acid halides of these.

Among these compounds, the monomer (B-1) having an acidic group is preferably a phosphoric acid group- or pyrophosphoric acid group-containing (meth)acrylic monomer, more particularly a phosphoric acid group-containing (meth)acrylic monomer, because these monomers develop superior adhesive properties to tooth structure. In view of the ability to exhibit high adhesive properties by showing high demineralization in the absence of an organic solvent, the monomer (B-1) having an acidic group is even more preferably a divalent phosphoric acid group-containing (meth)acrylic monomer having a C6 to C20 alkyl or alkylene group as a backbone within the molecule, particularly preferably a divalent phosphoric acid group-containing (meth)acrylic monomer having a C8 to C12 alkylene group as a backbone within the molecule (e.g., 10-methacryloyloxydecyl dihydrogen phosphate).

The monomer (B-1) having an acidic group may be used alone, or two or more thereof may be used in combination. Excessively high or low contents of monomer (B-1) having an acidic group may result in a decrease of adhesive properties. In this respect, the content of monomer (B-1) having an acidic group is preferably 1 to 50 parts by mass, more preferably 3 to 40 parts by mass, even more preferably 5 to 30 parts by mass with respect to total 100 parts by mass of the compound (A) having a weight-average molecular weight of 2,000 or more, and the monomer (B) in the dental composition.

### · Hydrophobic Monomer (B-2) Having no Acidic Group

The hydrophobic monomer (B-2) having no acidic group (hereinafter, referred to as "hydrophobic monomer (B-2)") improves the mechanical strength, ease of handling, and other properties of the dental composition. Preferably, the hydrophobic monomer (B-2) is a radical monomer having a polymerizable group, with no acidic group. In view of ease of radical polymerization, the polymerizable group is more preferably a (meth)acryl group, and/or a (meth)acrylamide group. A hydrophobic monomer (B-2) means a monomer that does not have an acidic group, and that has a solubility at 25°C of less than 10 mass% in water. Examples include crosslinkable monomers such as aromatic compound-based bifunctional monomers, aliphatic compound-based bifunctional monomers, and tri- and higher-functional monomers.

Examples of the aromatic compound-based bifunctional monomers include 2,2-bis((meth)acryloyloxyphenyl)propane, 2,2-bis[4-(3-(meth)acryloyloxy-2-hydroxypropoxy)phenyl]propane, 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, and 2,2-bis(4-(meth)acryloyloxyisopropoxyphenyl)propane. Preferred are 2,2-bis[4-(3-methacryloyloxy-2-hydroxypropoxy)phenyl]propane (commonly known as "Bis-GMA"), 2,2-bis(4-(meth)acryloyloxyethoxyphenyl)propane, 2,2-bis(4-methacryloyloxypolyethoxyphenyl)propane (average number of moles of ethoxy group added is 2.6; commonly known as "D-2.6E"), 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, and 2,2-bis(4-(meth)acryloyloxypentaethoxyphenyl)propane.

Examples of the aliphatic compound-based bifunctional monomers include glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 1,2-bis(3-methacryloyloxy-2-hydroxypropoxy)ethane, 2,2,4-trimethylhexamethylenebis(2-carbamoyloxyethyl)di(meth)acrylate, N-methacryloyloxyethylacrylamide, and N-methacryloyloxypropylamide. Preferred are triethylene glycol diacrylate, triethylene glycol dimethacrylate (commonly known as "3G"), neopentyl glycol di(meth)acrylate, 1,2-bis(3-methacryloyloxy-2-hydroxypropoxy)ethane, 2,2,4-trimethylhexamethylenebis(2-carbamoyloxyethyl)dimethacrylate (commonly known as "UDMA"), 1,10-decanediol dimethacrylate (commonly known as "DD"), 2,2,4-trimethylhexamethylenebis(2-carbamoyloxyethyl)dimethacrylate, and N-methacryloyloxyethylacrylamide (commonly known as "MAEA").

Examples of the tri- and higher-functional monomers include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol penta(meth)acrylate, N,N-(2,2,4-trimethylhexamethylene)bis[2-(aminocarboxy)propane-1,3-diol]tetra(meth)acr ylate, and 1,7-diacryloyloxy-2,2,6,6-tetra(meth)acryloyloxymethyl-4-oxaheptane. Preferred is N,N-(2,2,4-trimethylhexamethylene)bis[2-(aminocarboxy)propane-1,3-diol]tetramethacryl ate.

In view of mechanical strength and ease of handling, preferred for use as hydrophobic monomer (B-2) are aromatic compound-based bifunctional monomers and aliphatic compound-based bifunctional monomers. Preferred as aromatic compound-based bifunctional monomers are Bis-GMA and D-2.6E. Preferred as aliphatic compound-based bifunctional monomers are glycerol di(meth)acrylate, 3G, neopentyl glycol di(meth)acrylate, 1,6-hexanediol di(meth)acrylate, DD, 1,2-bis(3-methacryloyloxy-2-hydroxypropoxy)ethane, UDMA, and MAEA.

In view of initial adhesion to tooth structure, bond durability, and mechanical strength, the hydrophobic monomer (B-2) is more preferably Bis-GMA, D-2.6E, 3G, UDMA, DD, or MAEA, even more preferably D-2.6E, DD, or MAEA.

The hydrophobic monomer (B-2) may be contained alone, or two or more thereof may be used in combination. An excessively high content of hydrophobic monomer (B-2) may result in a decrease of adhesion due to a decrease of penetrability into the tooth structure, whereas the effect to improve mechanical strength may become insufficient with excessively low contents of hydrophobic monomer (B-2). In this respect, the content of hydrophobic monomer (B-2) is preferably 20 to 99 parts by mass, more preferably 40 to 95 parts by mass, even more preferably 60 to 92 parts by mass with respect to total 100 parts by mass of the compound (A) having a weight-average molecular weight of 2,000 or more, and the monomer (B) in the dental composition. In certain embodiments, in view of the mechanical strength, paste properties, and polymerization shrinkage stress reducing effect, the content of hydrophobic monomer (B-2) is preferably 1 mass% or more, more preferably 5 mass% or more, even more preferably 10 mass% or more in a total amount of the dental composition. In view of the mechanical strength, paste properties, and polymerization shrinkage stress reducing effect, the content of the hydrophobic monomer (B-2) is preferably 48 mass% or less, more preferably 40 mass% or less, even more preferably 35 mass% or less in a total amount of the dental composition.

### · Hydrophilic Monomer (B-3) Having no Acidic Group

Preferably, a dental composition of the present invention additionally comprises a hydrophilic monomer (B-3) having no acidic group (hereinafter, referred to as "hydrophilic monomer (B-3)"). The hydrophilic monomer (B-3) promotes penetration of the components of the dental composition into tooth structure. The hydrophilic monomer (B-3) itself also penetrates into tooth structure, and adheres to the organic component (collagen) in the tooth structure. The hydrophilic monomer (B-3) is preferably a radical monomer having a polymerizable group, with no acidic group. In view of ease of radical polymerization, the polymerizable group is more preferably a (meth)acryl group and/or a (meth)acrylamide group. A hydrophilic monomer (B-3) means a monomer that does not have an acidic group, and that has a solubility at 25°C of 10 mass% or more in water. Preferably, the hydrophilic monomer (B-3) is one having a solubility at 25°C of 30 mass% or more, more preferably one that can dissolve in water in any fractions at 25°C. The hydrophilic monomer (B-3) is preferably one having a hydrophilic group such as a hydroxyl group, an oxymethylene group, an oxyethylene group, an oxypropylene group, or an amide group. Examples of such monomers include hydrophilic monofunctional (meth)acrylate monomers such as 2-hydroxyethyl (meth)acrylate (HEMA), 3-hydroxypropyl (meth)acrylate, 2-hydroxypropyl (meth)acrylate, 1,3-dihydroxypropyl (meth)acrylate, 2,3-dihydroxypropyl (meth)acrylate, 2-((meth)acryloyloxy)ethyltrimethylammonium chloride, and polyethylene glycol di(meth)acrylate (number of oxyethylene groups is 9 or more); and hydrophilic monofunctional (meth)acrylamide monomers such as N-methylol(meth)acrylamide, N-hydroxyethyl(meth)acrylamide, N,N-bis(2-hydroxyethyl)(meth)acrylamide, N-methoxymethyl(meth)acrylamide, N-ethoxymethyl(meth)acrylamide, diacetone(meth)acrylamide, 4-(meth)acryloylmorpholine, N-trihydroxymethyl-N-methyl(meth)acrylamide, N,N-dimethylacrylamide, and N,N-diethylacrylamide.

In view of adhesive properties to tooth structure, preferred as hydrophilic monomer (B-3) are 2-hydroxyethyl (meth)acrylate, 2,3-dihydroxypropyl (meth)acrylate, and hydrophilic monofunctional (meth)acrylamide monomers. More preferred are 2-hydroxyethyl (meth)acrylate, N,N-dimethylacrylamide, and N,N-diethylacrylamide. The hydrophilic monomer (B-3) may be contained alone, or two or more thereof may be used in combination.

The presence of the hydrophilic monomer (B-3) in the present invention is expected to have an effect on improving penetration into tooth structure. However, the mechanical strength may decrease when the content of hydrophilic monomer (B-3) is excessively high. It is therefore preferable that the content of hydrophilic monomer (B-3) be 0 to 50 parts by mass, more preferably 0 to 40 parts by mass, even more preferably 0 to 30 parts by mass with respect to total 100 parts by mass of the compound (A) having a weight-average molecular weight of 2,000 or more, and the monomer (B) in the dental composition. The content of hydrophilic monomer (B-3) may be 0 part by mass.

### [Polymerization Initiator (C)]

The polymerization initiator (C) can be broadly classified into photopolymerization initiator and chemical polymerization initiator, with the photopolymerization initiator further divided into water-soluble photopolymerization initiator (C-1) and water-insoluble photopolymerization initiator (C-2). The polymerization initiator (C) may be solely a water-soluble photopolymerization initiator (C-1) or a water-insoluble photopolymerization initiator (C-2), or a combination of water-soluble photopolymerization initiator (C-1) and water-insoluble photopolymerization initiator (C-2).

### · Water-Soluble Photopolymerization Initiator (C-1)

The water-soluble photopolymerization initiator (C-1) can achieve high bond strength by improving the polymerization curability at the hydrophilic tooth interface. The water-soluble photopolymerization initiator (C-1) has a solubility in water of 1.0 mass% or more, preferably 1.5 mass% or more, more preferably 2.0 mass% or more, even more preferably 2.5 mass% or more at 25°C. When the solubility is less than 1.0 mass%, the water-soluble photopolymerization initiator (C-1) does not sufficiently dissolve in water in tooth structure, and fails to sufficiently promote polymerization at the bond interface.

Examples of the water-soluble photopolymerization initiator (C-1) include water-soluble acylphosphine oxides, water-soluble thioxanthones, and α-hydroxyalkylacetophenones. The α-hydroxyalkylacetophenones may be, for example, compounds having a (poly)ethylene glycol chain introduced into the hydroxyl group(s) of 1-[4-(2-hydroxyethoxy)-phenyl]-2-hydroxy-2-methyl-1-propan-1-one, compounds having a (poly)ethylene glycol chain introduced into the hydroxyl group and/or phenyl group of 1-hydroxycyclohexyl phenyl ketone, compounds having -OCH₂COO⁻Na⁺ introduced into the phenyl group of 1-hydroxycyclohexyl phenyl ketone, compounds having a (poly)ethylene glycol chain introduced into the hydroxyl group and/or phenyl group of 2-hydroxy-2-methyl-1-phenylpropan-1-one, and compounds having -OCH₂COO⁻Na⁺ introduced into the phenyl group of 2-hydroxy-2-methyl-1-phenylpropan-1-one. Other examples of the water-soluble photopolymerization initiator (C-1) include quaternary ammonium salt compounds prepared by quaternization of the amino group of α-aminoalkylphenones, such as 2-methyl-1[4-(methylthio)phenyl]-2-morpholinopropan-1-one, and 2-benzyl-2-dimethylamino-1-(4-morpholinophenyl)butanone-1.

The water-soluble thioxanthones may be any of, for example, 2-hydroxy-3-(9-oxo-9H-thioxanthen-4-yloxy)-N,N,N-trimethyl-1-propaneaminium chloride, 2-hydroxy-3-(1-methyl-9-oxo-9H-thioxanthen-4-yloxy)-N,N,N-trimethyl-1-propaneaminiu m chloride, 2-hydroxy-3-(9-oxo-9H-thioxanthen-2-yloxy)-N,N,N-trimethyl-1-propaneaminium chloride, 2-hydroxy-3-(3,4-dimethyl-9-oxo-9H-thioxanthen-2-yloxy)-N,N,N-trimethyl-1-propaneam i nium chloride, 2-hydroxy-3-(3,4-dimethyl-9H-thioxanthen-2-yloxy)-N,N,N-trimethyl-1-propaneaminium chloride, and 2-hydroxy-3-(1,3,4-trimethyl-9-oxo-9H-thioxanthen-2-yloxy)-N,N,N-trimethyl-1-propanea minium chloride.

The water-soluble acylphosphine oxides may be, for example, acylphosphine oxides represented by the following general formula (1) or (2).

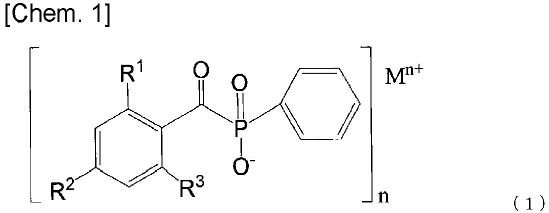

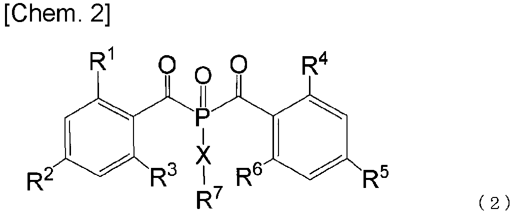

In the formulae, R¹, R², R³, R⁴, R⁵, and R⁶ are each independently a C1 to C4 linear or branched alkyl group or a halogen atom, M is a hydrogen ion, an alkali metal ion, an alkali earth metal ion, a magnesium ion, a pyridinium ion (the pyridine ring may have a substituent), or an ammonium ion represented by HN⁺R⁸R⁹R¹⁰ (where R⁸, R⁹, and R¹⁰ are each independently an organic group or a hydrogen atom), n is 1 or 2, X is a C1 to C4 linear or branched alkylene group, R⁷ represents -CH(CH₃)COO(C₂H₄O)ₚCH₃, where p is an integer of 1 to 1,000.

The alkyl group represented by R¹, R², R³, R⁴, R⁵, and R⁶ is not particularly limited, as long as it is C1 to C4 linear or branched alkyl. Examples include methyl, ethyl, n-propyl, isopropyl, n-butyl, isobutyl, sec-butyl, 2-methylpropyl, and tert-butyl. The alkyl group represented by R¹, R², R³, R⁴, R⁵, and R⁶ is preferably a C1 to C3 linear alkyl group, more preferably methyl or ethyl, even more preferably methyl. Examples of the alkylene group represented by X include a methylene group, an ethylene group, an n-propylene group, an isopropylene group, and an n-butylene group. The alkylene group represented by X is preferably a C1 to C3 linear alkylene group, more preferably a methylene group or an ethylene group, even more preferably a methylene group.

Examples of the substituent of the pyridine ring when M is a pyridinium ion include halogen atoms (a fluorine atom, a chlorine atom, a bromine atom, an iodine atom), a carboxy group, a C2 to C6 linear or branched acyl group, a C1 to C6 linear or branched alkyl group, and a C1 to C6 linear or branched alkoxy group. Preferably, M is an alkali metal ion, an alkali earth metal ion, a magnesium ion, a pyridinium ion (the pyridine ring may have a substituent), or an ammonium ion represented by HN⁺R⁸R⁹R¹⁰ (the symbols have the same meaning as above). Examples of the alkali metal ion include a lithium ion, a sodium ion, a potassium ion, a rubidium ion, and a cesium ion. Examples of the alkali earth metal ion include a calcium ion, a strontium ion, a barium ion, and a radium ion. The organic group represented by R⁸, R⁹, and R¹⁰ may be the same group exemplified above for the substituent of the pyridine ring (excluding a halogen atom).

In view of storage stability and shade stability in the composition, particularly preferred are compounds in which R¹, R², R³, R⁴, R⁵, and R⁶ are all methyl groups. Examples of Mⁿ⁺ include Li⁺, Na⁺, K⁺, Ca²⁺, Mg²⁺, and ammonium ions derived from various amines. Examples of the amines include ammonia, trimethylamine, diethylamine, dimethylaniline, ethylenediamine, triethanolamine, N,N-dimethylaminomethacrylate, N,N-dimethylaminobenzoic acid and alkyl esters thereof, N,N-diethylaminobenzoic acid and alkyl esters thereof, and N,N-bis(2-hydroxyethyl)-p-toluidine. In view of adhesive properties, p in R⁷ is preferably 1 or more, more preferably 2 or more, even more preferably 3 or more, particularly preferably 4 or more, and is preferably 1,000 or less, more preferably 100 or less, even more preferably 75 or less, particularly preferably 50 or less.

Particularly preferably, the water-soluble acylphosphine oxides are compounds represented by general formula (1) and in which M is Li, and compounds represented by general formula (2) and in which the moiety corresponding to the group represented by R⁷ is synthesized from polyethylene glycol methylether methacrylate having a molecular weight of 950.

The water-soluble acylphosphine oxides having such structures can be synthesized according to known methods, and some are available as commercially available products. For example, the methods disclosed in JP S57-197289 A and WO2014/095724 can be used for the synthesis of the water-soluble acylphosphine oxides. The water-soluble photopolymerization initiator (C-1) may be used alone, or two or more thereof may be used in combination.

The water-soluble photopolymerization initiator (C-1) may be dissolved in the dental composition, or may be dispersed in the form of a powder in the composition, provided that the water-soluble photopolymerization initiator (C-1) can dissolve in water at the surface of the tooth structure (moist body), and can selectively increase the polymerization curability at the bond interface and inside the resin-impregnated layer.

When the water-soluble photopolymerization initiator (C-1) is dispersed in the composition in the form of a powder, the average particle diameter is preferably 500 µm or less, more preferably 100 µm or less, even more preferably 50 µm or less because the water-soluble photopolymerization initiator (C-1) is more likely to settle when the average particle diameter is excessively large. The average particle diameter is preferably 0.01 µm or more because the specific surface area of the powder overly increases, and the amount of powder that can be dispersed in the composition decreases when the average particle diameter is excessively small. Taken together, the average particle diameter of water-soluble photopolymerization initiator (C-1) preferably ranges from 0.01 to 500 µm, more preferably 0.01 to 100 µm, even more preferably 0.01 to 50 µm.

The average particle diameter of a powder of water-soluble photopolymerization initiator (C-1) can be determined by taking an electron micrograph of at least 100 particles, and calculating the volume average particle diameter from the captured image after an image analysis performed with image-analyzing particle size distribution measurement software (Mac-View, manufactured by Mountech Co., Ltd.).

The shape of the water-soluble photopolymerization initiator (C-1) when dispersed in the composition in the form of a powder is not particularly limited, and may be any of various shapes, including, for example, spherical, stylus, plate-like, and crushed shapes. The water-soluble photopolymerization initiator (C-1) can be prepared using a known method such as pulverization, freeze drying, or reprecipitation. In view of the average particle diameter of the powder obtained, freeze drying and reprecipitation are preferred, and freeze drying is more preferred.

In view of curability and other properties of the dental composition obtained, the content of water-soluble photopolymerization initiator (C-1) is preferably 0.01 to 20 parts by mass with respect to total 100 parts by mass of the compound (A) having a weight-average molecular weight of 2,000 or more, and the monomer (B) in the dental composition. In view of providing high initial adhesion and bond durability and reducing polymerization shrinkage stress, the content of water-soluble photopolymerization initiator (C-1) is more preferably 0.05 to 10 parts by mass, even more preferably 0.1 to 5 parts by mass. When the content of water-soluble photopolymerization initiator (C-1) is less than 0.01 parts by mass, polymerization may fail to sufficiently proceed at the bond interface, and the bond strength may decrease. When the content of water-soluble photopolymerization initiator (C-1) is more than 20 parts by mass, it may not be possible to obtain a sufficient bond strength when the water-soluble photopolymerization initiator (C-1) has low polymerization performance, in addition to making it difficult to sufficiently dissolve, disperse, or diffuse water-soluble photopolymerization initiator (C-1) in the dental composition.

### · Water-Insoluble Photopolymerization Initiator (C-2)

In view of curability, a dental composition of the present invention may comprise a water-insoluble photopolymerization initiator (C-2) having a solubility in water of less than 1.0 mass% at 25°C (hereinafter, referred to as "water-insoluble photopolymerization initiator (C-2)"), other than the water-soluble photopolymerization initiator (C-1). The water-insoluble photopolymerization initiator (C-2) used in the present invention may be a known photopolymerization initiator. The water-insoluble photopolymerization initiator (C-2) may be contained alone, or two or more thereof may be contained in combination.

Examples of the water-insoluble photopolymerization initiator (C-2) include (bis)acylphosphine oxides, thioxanthones, ketals, α-diketones, coumarins, anthraquinones, benzoin alkyl ether compounds, and α-aminoketone compounds, excluding those exemplified for water-soluble photopolymerization initiator (C-1).

Examples of acylphosphine oxides in the (bis)acylphosphine oxides include 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylethoxyphenylphosphine oxide, 2,3,5,6-tetramethylbenzoyldiphenylphosphine oxide, and benzoyl di(2,6-dimethylphenyl)phosphonate. Examples of bisacylphosphine oxides include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, and bis(2,5,6-trimethylbenzoyl)-2,4,4-trimethylpentylphosphine oxide.

Examples of the thioxanthones include thioxanthone, and 2-chlorothioxanthen-9-one.

Examples of the ketals include benzyl dimethyl ketal, and benzyl diethyl ketal.

Examples of the α-diketones include diacetyl, benzyl, dl-camphorquinone, 2,3-pentadione, 2,3-octadione, 9,10-phenanthrenequinone, 4,4'-oxybenzyl, and acenaphthenequinone. Particularly preferred is dl-camphorquinone for its maximum absorption wavelength occurring in the visible light region.

Examples of the coumarin compounds include compounds mentioned in JP H9-3109 A and JP H10-245525 A, including, for example, 3,3'-carbonylbis(7-diethylaminocoumarin), 3-(4-methoxybenzoyl)coumarin, 3-thienoylcoumarin, 3-benzoyl-5,7-dimethoxycoumarin, 3-benzoyl-7-methoxycoumarin, 3-benzoyl-6-methoxycoumarin, 3-benzoyl-8-methoxycoumarin, 3-benzoylcoumarin, 7-methoxy-3-(p-nitrobenzoyl)coumarin, 3-(p-nitrobenzoyl)coumarin, 3,5-carbonylbis(7-methoxycoumarin), 3-benzoyl-6-bromocoumarin, 3,3'-carbonylbiscoumarin, 3-benzoyl-7-dimethylaminocoumarin, 3-benzoylbenzo[f]coumarin, 3-carboxycoumarin, 3-carboxy-7-methoxycoumarin, 3-ethoxycarbonyl-6-methoxycoumarin, 3-ethoxycarbonyl-8-methoxycoumarin, 3-acetylbenzo[f]coumarin, 3-benzoyl-6-nitrocoumarin, 3-benzoyl-7-diethylaminocoumarin, 7-dimethylamino-3-(4-methoxybenzoyl)coumarin, 7-diethylamino-3-(4-methoxybenzoyl)coumarin, 7-diethylamino-3-(4-diethylamino)coumarin, 7-methoxy-3-(4-methoxybenzoyl)coumarin, 3-(4-nitrobenzoyl)benzo[f]coumarin, 3-(4-ethoxycinnamoyl)-7-methoxycoumarin, 3-(4-dimethylaminocinnamoyl)coumarin, 3-(4-diphenylaminocinnamoyl)coumarin, 3-[(3-dimethylbenzothiazol-2-ylidene)acetyl]coumarin, 3-[(1-methylnaphtho[1,2-d]thiazol-2-ylidene)acetyl]coumarin, 3,3'-carbonylbis(6-methoxycoumarin), 3,3'-carbonylbis(7-acetoxycoumarin), 3,3'-carbonylbis(7-dimethylaminocoumarin), 3-(2-benzothiazolyl)-7-(diethylamino)coumarin, 3-(2-benzothiazolyl)-7-(dibutylamino)coumarin, 3-(2-benzoimidazolyl)-7-(diethylamino)coumarin, 3-(2-benzothiazolyl)-7-(dioctylamino)coumarin, 3-acetyl-7-(dimethylamino)coumarin, 3,3'-carbonylbis(7-dibutylaminocoumarin), 3,3'-carbonyl-7-diethylaminocoumarin-7'-bis(butoxyethyl)aminocoumarin, 10-[3-[4-(dimethylamino)phenyl]-1-oxo-2-propenyl]-2,3,6,7-tetrahydro-1,1,7,7-tetramethy I-1H,5H,11H-[1]benzopyrrano[6,7,8-ij]quinolizin-11-one, and 10-(2-benzothiazolyl)-2,3,6,7-tetrahydro-1,1,7,7-tetramethyl-1H,5H,11H-[1]benzopyrrano [6,7,8-ij]quinolizin-11-one.

Particularly preferred among these coumarin compounds are 3,3'-carbonylbis(7-diethylaminocoumarin) and 3,3'-carbonylbis(7-dibutylaminocoumarin).

Examples of the anthraquinones include anthraquinone, 1-chloroanthraquinone, 2-chloroanthraquinone, 1-bromoanthraquinone, 1,2-benzanthraquinone, 1-methylanthraquinone, 2-ethylanthraquinone, and 1-hydroxyanthraquinone.

Examples of the benzoin alkyl ether compounds include benzoin methyl ether, benzoin ethyl ether, benzoin isopropyl ether, and benzoin isobutyl ether.

Examples of the α-aminoketone compounds include 2-methyl-1-[4-(methylthio)phenyl]-2-morpholinopropan-1-one.

The water-insoluble photopolymerization initiator (C-2) is preferably at least one selected from the group consisting of a (bis)acylphosphine oxide, an α-diketone, and a coumarin compound. In this way, a dental composition can be obtained that has desirable photocurability both in the visible light region and the near ultraviolet region, and that shows sufficient photocurability regardless of whether the light source used is a halogen lamp, a light emitting diode (LED), or a xenon lamp.

The content of water-insoluble photopolymerization initiator (C-2) is not particularly limited. However, in view of curability and other properties of the composition obtained, the content of water-insoluble photopolymerization initiator (C-2) preferably ranges from 0.01 to 10 parts by mass, more preferably 0.05 to 7 parts by mass, even more preferably 0.1 to 5 parts by mass with respect to total 100 parts by mass of the compound (A) having a weight-average molecular weight of 2,000 or more, and the monomer (B) in the dental composition. When the content of water-insoluble photopolymerization initiator (C-2) is more than 10 parts by mass, it may not be possible to obtain a sufficient bond strength when the polymerization initiator itself has low polymerization performance, in addition to raising a possibility of precipitation from the dental composition.

In the present invention, the mass ratio of water-soluble photopolymerization initiator (C-1) and water-insoluble photopolymerization initiator (C-2) [(C-1):(C-2)] is preferably 10:1 to 1:10, more preferably 7:1 to 1:7, even more preferably 5:1 to 1:5, particularly preferably 3:1 to 1:3. When the fraction of water-soluble photopolymerization initiator (C-1) in the mass ratio exceeds 10:1, the curability of the dental composition itself decreases, and the dental composition may have difficulty exhibiting high bond strength. When the fraction of water-insoluble photopolymerization initiator (C-2) in the mass ratio exceeds 1:10, the dental composition may have difficulty exhibiting high bond strength as a result of insufficient promotion of polymerization at the bond interface, though the curability of the dental composition itself increases.

### [Chemical Polymerization Initiator]

A dental composition of the present invention may additionally comprise a chemical polymerization initiator, for which an organic peroxide is preferred. The organic peroxide used as the chemical polymerization initiator is not particularly limited, and known organic peroxides may be used. Typical examples of such organic peroxides include ketoneperoxides, hydroperoxides, diacyl peroxides, dialkyl peroxides, peroxyketals, peroxyesters, and peroxy dicarbonates. Specific examples of these organic peroxides include those mentioned in WO2008/087977.

### [Filler (D)]

A dental composition of the present invention may further comprise a filler (D). In the present invention, the filler (D) can be broadly classified into organic fillers, inorganic fillers, and organic-inorganic composite fillers.

Examples of the materials of the organic fillers include polymethylmethacrylate, polyethylmethacrylate, a methyl methacrylate-ethyl methacrylate copolymer, crosslinked polymethylmethacrylate, crosslinked polyethylmethacrylate, polyamides, polyvinyl chloride, polystyrene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer. These may be used alone, or two or more thereof may be used as a mixture. The shape of the organic filler is not particularly limited, and the particle diameter of the filler may be appropriately selected for use.

Examples of the materials of the inorganic fillers include quartz, silica, alumina, silica-titania, silica-titania-barium oxide, silica-zirconia, silica-alumina, lanthanum glass, borosilicate glass, soda glass, barium glass, strontium glass, glass-ceramics, aluminosilicate glass, barium boroaluminosilicate glass, strontium boroaluminosilicate glass, fluoroaluminosilicate glass, calcium fluoroaluminosilicate glass, strontium fluoroaluminosilicate glass, barium fluoroaluminosilicate glass, strontium calcium fluoroaluminosilicate glass, ytterbium oxide, and silica-coated ytterbium fluoride. These may be used alone, or two or more thereof may be used as a mixture. The shape of the inorganic filler is not particularly limited, and the particle diameter of the filler may be appropriately selected for use. In view of considerations such as the ease of handling and mechanical strength of the composition obtained, the average particle diameter of the inorganic filler is preferably 0.001 to 50 µm, more preferably 0.001 to 10 µm, even more preferably 0.001 to 8 µm. When the inorganic filler is surface treated with a surface treatment agent, the average particle diameter of the inorganic filler means the average particle diameter before surface treatment.

The inorganic filler may be, for example, an irregularly shaped filler or a spherical filler. In view of improving the mechanical strength of the composition, the inorganic filler used is preferably a spherical filler. Another advantage of using a spherical filler is that the dental composition of the present invention, when used as a self-adhesive dental composite resin, can produce a composite resin having excellent surface gloss. Here, the spherical filler used in the present invention is a filler having an average uniformity of 0.6 or more as measured for round-shaped particles observed in a unit field of an electron micrograph of filler by dividing the diameter of a particle perpendicular to its maximum diameter by the maximum diameter. The spherical filler has an average particle diameter of preferably 0.05 to 5 µm. When the average particle diameter is less than 0.05 µm, the mechanical strength may decrease as a result of a decrease in the filling rate of the spherical filler in the composition. When the average particle diameter is more than 5 µm, the spherical filler has a reduced surface area, and the dental composition may fail to produce a cured product having high mechanical strength.

In order to adjust the flowability of the dental composition, the inorganic filler may be used after an optional surface treatment with a known surface treatment agent such as a silane coupling agent. Examples of the surface treatment agent include vinyltrimethoxysilane, vinyltriethoxysilane, vinyltrichlorosilane, vinyl tri(β-methoxyethoxy)silane, γ-methacryloyloxypropyltrimethoxysilane, 8-methacryloyloxyoctyltrimethoxysilane, 11-methacryloyloxyundecyltrimethoxysilane, γ-glycidoxypropyltrimethoxysilane, γ-mercaptopropyltrimethoxysilane, and γ-aminopropyltriethoxysilane.

The organic-inorganic composite filler used in the present invention is a filler obtained by adding a monomer compound to the inorganic filler, polymerizing the mixture in paste form, and pulverizing the polymerized filler. The organic-inorganic composite filler may be, for example, a TMPT filler (a filler obtained by mixing trimethylolpropane methacrylate and a silica filler, and pulverizing the mixture after polymerization). The shape of the organic-inorganic composite filler is not particularly limited, and may be determined by appropriately selecting the particle size of the filler. In view of properties such as the ease of handling and mechanical strength of the composition obtained, the organic-inorganic composite filler has an average particle diameter of preferably 0.001 to 50 µm, more preferably 0.001 to 10 µm.

In this specification, the average particle diameter of filler can be determined using a laser diffraction scattering method or by observing particles with an electron microscope. Specifically, a laser diffraction scattering method is more convenient for the measurement of particles of 0.1 µm or more, whereas electron microscopy is a more convenient method of particle size measurement for ultrafine particles of less than 0.1 µm. Here, 0.1 µm is a measured value by a laser diffraction scattering method.

As a specific example of a laser diffraction scattering method, the particle size may be measured by volume using, for example, a laser diffraction particle size distribution analyzer (SALD-2300, manufactured by Shimadzu Corporation) with a 0.2% sodium hexametaphosphate aqueous solution used as dispersion medium.

In electron microscopy, for example, particles may be photographed with an electron microscope (Model S-4000, manufactured by Hitachi), and the size of particles (at least 200 particles) observed in a unit field of the captured image may be measured using image-analyzing particle-size-distribution measurement software (Mac-View, manufactured by Mountech Co., Ltd.). Here, the particle diameter is determined as an arithmetic mean value of the maximum and minimum lengths of particles, and the average primary particle diameter is calculated from the number of particles and the particle diameter.

The filler (D) used in the present invention may be a mixture or combination of two or more kinds of fillers of different materials having different particle size distributions and different forms. Unintended inclusion of non-filler particles as impurities is acceptable to such an extent that it is not detrimental to the effects of the present invention.

The content of the filler (D) used in the present invention is not particularly limited. Preferably, the dental composition comprises 0 to 2,000 parts by mass of filler (D) with respect to total 100 parts by mass of the compound (A) having a weight-average molecular weight of 2,000 or more, and the monomer (B) in the dental composition. In certain embodiments, in view of the present invention producing more superior effects such as mechanical strength, the content of filler (D) is preferably 50 mass% or more, more preferably 55 mass% or more, even more preferably 60 mass% or more in a total amount of the dental composition. In view of the present invention producing more superior effects such as mechanical strength, the content of filler (D) is preferably 98 mass% or less, more preferably 94 mass% or less, even more preferably 88 mass% or less in a total amount of the dental composition. Because the preferred content of filler (D) greatly differs in different embodiments, the preferred content of filler (D) suited for each embodiment is presented below along with descriptions of specific embodiments of a dental composition of the present invention.

### [Polymerization Accelerator (E)]

In certain embodiments, a polymerization accelerator (E) is used with the water-insoluble photopolymerization initiator (C-2) and/or the chemical polymerization initiator. Examples of the polymerization accelerator (E) used in the present invention include amines, sulfinic acid and salts thereof, borate compounds, derivatives of barbituric acid, triazine compounds, copper compounds, tin compounds, vanadium compounds, halogen compounds, aldehydes, thiol compounds, sulfites, bisulfites, and thiourea compounds.

The amines used as polymerization accelerator (E) can be categorized into aliphatic amines and aromatic amines. Examples of the aliphatic amines include primary aliphatic amines such as n-butylamine, n-hexylamine, and n-octylamine; secondary aliphatic amines such as diisopropylamine, dibutylamine, and N-methylethanolamine; and tertiary aliphatic amines such as N-methyldiethanolamine, N-ethyldiethanolamine, N-n-butyldiethanolamine, N-lauryldiethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, triethanolamine trimethacrylate, triethanolamine, trimethylamine, triethylamine, and tributylamine. In view of the curability and storage stability of the dental composition, preferred for use are tertiary aliphatic amines, and N-methyldiethanolamine and triethanolamine are more preferred.

Examples of the aromatic amines include N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N, N-bis(2-hydroxyethyl)-3,5-diisopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, ethyl 4-(N,N-dimethylamino)benzoate, methyl 4-(N,N-dimethylamino)benzoate, propyl 4-(N,N-dimethylamino)benzoate, n-butoxyethyl 4-(N,N-dimethylamino)benzoate, 2-(methacryloyloxy)ethyl 4-(N,N-dimethylamino)benzoate, 4-(N,N-dimethylamino)benzophenone, and butyl 4-(N,N-dimethylamino)benzoate. In view of the ability to impart superior curability to the dental composition, it is preferable to use at least one selected from the group consisting of N,N-bis(2-hydroxyethyl)-p-toluidine, ethyl 4-(N,N-dimethylamino)benzoate, n-butoxyethyl 4-(N,N-dimethylamino)benzoate, and 4-(N,N-dimethylamino)benzophenone.

Specific examples of sulfinic acid and salts thereof, borate compounds, derivatives of barbituric acid, triazine compounds, copper compounds, tin compounds, vanadium compounds, halogen compounds, aldehydes, thiol compounds, sulfites, bisulfites, and thiourea compounds include those mentioned in WO2008/087977.

The polymerization accelerator (E) may be contained alone, or two or more thereof may be contained in combination. The content of the polymerization accelerator (E) used in the present invention is not particularly limited. However, in view of curability and other properties of the dental composition obtained, the content of polymerization accelerator (E) is preferably 0.001 parts or more by mass, more preferably 0.01 parts or more by mass, even more preferably 0.1 parts or more by mass, and is preferably 30 parts or less by mass, more preferably 10 parts or less by mass, even more preferably 5 parts or less by mass with respect to total 100 parts by mass of the compound (A) having a weight-average molecular weight of 2,000 or more, and the monomer (B) in the dental composition. When the content of polymerization accelerator (E) is less than 0.001 parts by mass, polymerization may fail to proceed sufficiently, and this may lead to a decrease of adhesive properties. In this respect, the content of polymerization accelerator (E) is more preferably 0.05 parts or more by mass. When the content of polymerization accelerator (E) is more than 30 parts by mass, it may not be possible to obtain sufficient adhesive properties when the polymerization initiator itself has low polymerization performance, in addition to raising a possibility of precipitation from the dental composition. In this respect, the content of polymerization accelerator (E) is more preferably 20 parts or less by mass.

### [Fluorine-Ion Releasing Substance]

A dental composition of the present invention may further comprise a fluorine-ion releasing substance. By containing a fluorine-ion releasing substance, the dental composition produced can impart acid resistance to tooth structure. Examples of the fluorine-ion releasing substance include metal fluorides such as sodium fluoride, potassium fluoride, sodium monofluorophosphate, lithium fluoride, and ytterbium fluoride. The fluorine-ion releasing substance may be contained alone, or two or more thereof may be contained in combination.

The dental composition may additionally comprise additives such as pH adjusters, polymerization inhibitors, thickeners, colorants, fluorescent agents, fragrances, and cross-linking agents (for example, polyvalent metal ion releasing components), provided that such additives do not hinder the effects of the present invention. These may be used alone, or two or more thereof may be used in combination. A dental composition of the present invention may also comprise anti-microbial substances such as cetylpyridinium chloride, benzalkonium chloride, (meth)acryloyloxydodecylpyridinium bromide, (meth)acryloyloxyhexadecylpyridinium chloride, (meth)acryloyloxydecylammonium chloride, and triclosan. A dental composition of the present invention may comprise a known dye or pigment as a colorant.

A dental composition of the present invention may comprise a solvent, depending on use. The solvent may be, for example, water or an organic solvent. The organic solvent may be any known organic solvent. Examples of such solvents include alcohol solvents (such as methanol, ethanol, 1-propanol, and 2-propanol), acetone, methyl ethyl ketone, tetrahydrofuran, diethyl ether, diisopropyl ether, hexane, toluene, chloroform, ethyl acetate, and butyl acetate. Preferred are alcohol solvents. In an embodiment using an organic solvent, the content of organic solvent is preferably 1 to 2,000 parts by mass, more preferably 2 to 1,000 parts by mass, even more preferably 3 to 500 parts by mass with respect to total 100 parts by mass of the compound (A) having a weight-average molecular weight of 2,000 or more, and the monomer (B). For example, when used as a dental composite resin (particularly preferably, a self-adhesive dental composite resin) or a dental cement, a dental composition of the present invention may be a dental composition that does not comprise a solvent. However, inclusion of trace amounts of water or organic solvent (for example, 3 mass% or less with respect to the composition) is acceptable, provided that it does not cause problems such as improper curing or delayed cure. Some commercially available products are sold as products containing water or organic solvent (for example, colloidal silica), depending on the components. Such products can be used for the preparation of a dental composition of the present invention after removing water or organic solvent to the acceptable limits.

The components (for example, a prepolymer (oligomer) different from the compound (A) having a weight-average molecular weight of 2,000 or more) of a dental composition of the present invention other than the compound (A) having a weight-average molecular weight of 2,000 or more, the monomer (B), the polymerization initiator (C), the filler (D), the polymerization accelerator (E), and polymerization inhibitors and colorants are preferably less than 0.1 parts by mass, more preferably less than 0.01 parts by mass, even more preferably less than 0.001 parts by mass in 100 parts by mass of the dental composition. A dental composition of the present invention is preferably one having a polymerization shrinkage stress of less than 10 MPa, more preferably less than 9.5 MPa, even more preferably less than 9.0 MPa. The method of measurement of polymerization shrinkage stress is as described in the EXAMPLES section below.

A dental composition of the present invention can be used for dental treatment as, for example, a dental composite resin (particularly preferably, a self-adhesive dental composite resin), a dental bonding material, a dental cement, a pit and fissure sealant, a loose tooth fixing material, an abutment construction material, or an orthodontic bonding material. Particularly, a dental composition of the present invention is suited as a dental composite resin (including a self-adhesive dental composite resin) or a dental cement. In such applications, a dental composition of the present invention may be provided in two bottles or two pastes of divided components, or may be provided in one bottle or one paste. The following describes specific embodiments of different uses of a dental composition of the present invention.

### <Self-Adhesive Dental Composite Resin>

A preferred embodiment of a dental composition of the present invention is, for example, a self-adhesive dental composite resin. A self-adhesive dental composite resin formed of a dental composition of the present invention comprises a monomer (B-1) having an acidic group. When a dental composition of the present invention is used as a self-adhesive dental composite resin, it is preferable that a dental composition of the present invention comprise a compound (A) having a weight-average molecular weight of 2,000 or more, a monomer (B), a polymerization initiator (C), a filler (D), and a polymerization accelerator (E), and that the monomer (B) comprise a monomer (B-1) having an acidic group, a hydrophobic monomer (B-2) having no acidic group, and a hydrophilic monomer (B-3) having no acidic group. The polymerization initiator (C) is preferably a photopolymerization initiator. More preferably, the polymerization initiator (C) comprises a water-soluble photopolymerization initiator (C-1) and a water-insoluble photopolymerization initiator (C-2). A pretreatment agent may be used when a dental composition of the present invention is used as a self-adhesive dental composite resin. However, because a self-adhesive dental composite resin has self-adhesive properties, a pretreatment agent is not essential, and it is not necessarily required to use a pretreatment agent. In this way, a dental composition of the present invention can be solely used as a self-adhesive dental composite resin, with no pretreatment agent.

The content of each component in the self-adhesive dental composite resin is such that the dental composition comprises preferably 0.1 to 50 parts by mass of compound (A) having a weight-average molecular weight of 2,000 or more, 1 to 50 parts by mass of monomer (B-1) having an acidic group, 20 to 99 parts by mass of hydrophobic monomer (B-2) having no acidic group, and 0 to 50 parts by mass of hydrophilic monomer (B-3) having no acidic group, more preferably 0.5 to 40 parts by mass of compound (A) having a weight-average molecular weight of 2,000 or more, 1 to 40 parts by mass of monomer (B-1) having an acidic group, 40 to 99 parts by mass of hydrophobic monomer (B-2) having no acidic group, and 0 to 40 parts by mass of hydrophilic monomer (B-3) having no acidic group, even more preferably 1 to 35 parts by mass of compound (A) having a weight-average molecular weight of 2,000 or more, 1 to 30 parts by mass of monomer (B-1) having an acidic group, 60 to 99 parts by mass of hydrophobic monomer (B-2) having no acidic group, and 0 to 30 parts by mass of hydrophilic monomer (B-3) having no acidic group with respect to total 100 parts by mass of the compound (A) having a weight-average molecular weight of 2,000 or more, and the monomer (B) in the dental composition. The dental composition preferably comprises 0.001 to 30 parts by mass of polymerization initiator (C), 50 to 2,000 parts by mass of filler (D), and 0.001 to 20 parts by mass of polymerization accelerator (E), and more preferably comprises 0.05 to 10 parts by mass of polymerization initiator (C), 100 to 1,500 parts by mass of filler (D), and 0.05 to 10 parts by mass of polymerization accelerator (E) with respect to total 100 parts by mass of the compound (A) having a weight-average molecular weight of 2,000 or more, and the monomer (B). The dental composition may or may not contain a hydrophilic monomer (B-3) when used as a self-adhesive dental composite resin.

### <Dental Composite Resin (Excluding Self-Adhesive Dental Composite Resin)>

Another preferred embodiment of a dental composition of the present invention is, for example, a dental composite resin. A dental composite resin formed of a dental composition of the present invention does not comprise a monomer (B-1) having an acidic group. When used as a dental composite resin, a dental composition of the present invention preferably comprises a compound (A) having a weight-average molecular weight of 2,000 or more, a hydrophobic monomer (B-2) having no acidic group, a hydrophilic monomer (B-3) having no acidic group, a polymerization initiator (C), a filler (D), and a polymerization accelerator (E). The polymerization initiator (C) preferably comprises a photopolymerization initiator. More preferably, the polymerization initiator (C) comprises a water-soluble photopolymerization initiator (C-1) and a water-insoluble photopolymerization initiator (C-2). When a dental composition of the present invention is used as a dental composite resin, the use of a dental bonding material or a pretreatment agent is essential.

The content of each component in the dental composite resin is such that the dental composition comprises preferably 0.1 to 50 parts by mass of compound (A) having a weight-average molecular weight of 2,000 or more, 50 to 99 parts by mass of hydrophobic monomer (B-2) having no acidic group, and 0 to 40 parts by mass of hydrophilic monomer (B-3) having no acidic group, more preferably 0.5 to 40 parts by mass of compound (A) having a weight-average molecular weight of 2,000 or more, 60 to 99 parts by mass of hydrophobic monomer (B-2) having no acidic group, and 0 to 30 parts by mass of hydrophilic monomer (B-3) having no acidic group, even more preferably 1 to 35 parts by mass of compound (A) having a weight-average molecular weight of 2,000 or more, 70 to 99 parts by mass of hydrophobic monomer (B-2) having no acidic group, and 0 to 20 parts by mass of hydrophilic monomer (B-3) having no acidic group with respect to total 100 parts by mass of the compound (A) having a weight-average molecular weight of 2,000 or more, and the monomer (B) in the dental composition. The dental composition comprises preferably 0.001 to 30 parts by mass of polymerization initiator (C), 50 to 2,000 parts by mass of filler (D), and 0.001 to 20 parts by mass of polymerization accelerator (E), more preferably 0.05 to 10 parts by mass of polymerization initiator (C), 100 to 1,500 parts by mass of filler (D), and 0.05 to 10 parts by mass of polymerization accelerator (E) with respect to total 100 parts by mass of the compound (A) having a weight-average molecular weight of 2,000 or more, and the monomer (B). The dental composition may or may not contain a hydrophilic monomer (B-3) when used as a dental composite resin.

### <Dental Cement>

Another preferred embodiment of a dental composition of the present invention is, for example, a dental cement. Preferred examples of the dental cement include resin cements, glass ionomer cements, and resin-reinforced glass ionomer cements. The dental cement may use, for example, a self-etching primer as a pretreatment agent. When used as a dental cement, it is preferable that a dental composition of the present invention comprise a compound (A) having a weight-average molecular weight of 2,000 or more, a monomer (B), a polymerization initiator (C), a filler (D), and a polymerization accelerator (E), and that the monomer (B) comprise a monomer (B-1) having an acidic group, a hydrophobic monomer (B-2) having no acidic group, and a hydrophilic monomer (B-3) having no acidic group. The polymerization initiator (C) preferably comprises a chemical polymerization initiator. More preferably, a chemical polymerization initiator and a photopolymerization initiator are used in combination. Preferably, the photopolymerization initiator is a combination of a water-soluble photopolymerization initiator (C-1) and a water-insoluble photopolymerization initiator (C-2).

The content of each component in the dental cement is such that the dental composition comprises preferably 0.1 to 50 parts by mass of compound (A) having a weight-average molecular weight of 2,000 or more, 0 to 50 parts by mass of monomer (B-1) having an acidic group, 50 to 99 parts by mass of hydrophobic monomer (B-2) having no acidic group, and 0 to 50 parts by mass of hydrophilic monomer (B-3) having no acidic group, more preferably 0.5 to 40 parts by mass of compound (A) having a weight-average molecular weight of 2,000 or more, 0 to 40 parts by mass of monomer (B-1) having an acidic group, 60 to 99 parts by mass of hydrophobic monomer (B-2) having no acidic group, and 0 to 40 parts by mass of hydrophilic monomer (B-3) having no acidic group, even more preferably 1 to 35 parts by mass of compound (A) having a weight-average molecular weight of 2,000 or more, 0 to 30 parts by mass of monomer (B-1) having an acidic group, 70 to 99 parts by mass of hydrophobic monomer (B-2) having no acidic group, and 0 to 30 parts by mass of hydrophilic monomer (B-3) having no acidic group with respect to total 100 parts by mass of the compound (A) having a weight-average molecular weight of 2,000 or more, and the monomer (B) in the dental composition. The dental composition comprises preferably 0.001 to 30 parts by mass of polymerization initiator (C), 50 to 2,000 parts by mass of filler (D), and 0.001 to 20 parts by mass of polymerization accelerator (E), more preferably 0.05 to 10 parts by mass of polymerization initiator (C), 100 to 1,500 parts by mass of filler (D), and 0.05 to 10 parts by mass of polymerization accelerator (E) with respect to total 100 parts by mass of the compound (A) having a weight-average molecular weight of 2,000 or more, and the monomer (B). The dental composition may or may not contain a hydrophilic monomer (B-3). The dental composition is not required to contain a monomer (B-1) having an acidic group when the dental composition is of a type that uses a pretreatment agent.

In all of these preferred embodiments as a self-adhesive dental composite resin, a dental composite resin, and a dental cement, the content of each component may be appropriately varied, and changes such as addition and deletion of any component may be made following the above descriptions of the present specification.

The present invention encompasses embodiments combining the foregoing features, provided that the present invention can exhibit its effects with such combinations made in various forms within the technical idea of the present invention.

### EXAMPLES

The following describes the present invention in greater detail by way of Examples. However, the present invention is not limited by the following EXAMPLES. It should also be noted that the combinations of the features described in the EXAMPLES below do not necessarily represent all the means necessary for solving the problems identified in the present invention. The components used in the following Examples and Comparative Examples, and the abbreviations and the structures of these components are presented below, along with the test methods used.

### [Compound (A) Having Weight-Average Molecular Weight of 2,000 or More]

UN-7600: urethane acrylate (manufactured by Negami Chemical Industrial Co., Ltd.; viscosity: 1,100,000 cps/25°C, weight-average molecular weight (Mw): 11,500, glass transition region: -51°C to 60°C, glass transition temperature (Tg): -42°C and 44.6°C, polyester skeleton-containing urethane acrylate, number of polymerizable groups (acryl groups): 2, weight-average molecular weight per polymerizable group: 5,750)

### <Synthesis Example 1> [Production of Compound 1]

(1) To a 5 L four-neck flask equipped with a stirrer, a thermostat, a thermometer, and a condenser were added 125 g of isophorone diisocyanate and 0.4 g of di-n-butyltin dilaurate, and the mixture was heated to 70°C under stirring.
(2) Separately, 850 g of polyester polyol (Kuraray Polyol^{®} P-5010 manufactured by Kuraray Co., Ltd.; a polymer of adipic acid and 3-methyl-1,5-pentanediol, weight-average molecular weight Mw: 5,000) was added into a dropping funnel having a side tube. The solution in the dropping funnel was then dropped in the flask of (1). Here, the solution was dropped at a constant rate over the course of 4 hours while stirring the solution in the flask (1), with the inside temperature of the flask held at 65 to 75°C. After the completion of dropping, the mixture was stirred at the same temperature for 24 hours to allow reaction.
(3) Thereafter, a solution prepared by uniformly dissolving 75 g of 2-hydroxyethylacrylate and 0.4 g of hydroquinone monomethyl ether added into a different dropping funnel was dropped at a constant rate over the course of 2 hours with the inside temperature of the flask held at 55 to 65°C. The mixture was allowed to react for 4 hours while maintaining the solution temperature in the flask at 70 to 80°C. This yielded a compound 1.
Compound 1: (viscosity: 900,000 cps/25°C, weight-average molecular weight (Mw): 13,000, glass transition region: -35°C to 55°C, glass transition temperature: -25°C and 35°C, number of polymerizable groups (acryl groups): 2, weight-average molecular weight per polymerizable group: 6,500)

### <Synthesis Example 2> [Production of Compound 2]

(1) To a 5 L four-neck flask equipped with a stirrer, a thermostat, a thermometer, and a condenser were added 882 g of polyester polyol (Kuraray Polyol^{®} P-2010 manufactured by Kuraray Co., Ltd.; a polymer of adipic acid and 3-methyl-1,5-pentanediol, weight-average molecular weight Mw: 2,000), 212 g of a monoadduct (VESTANATEP-DC1241, manufactured by Evonik Industries) as a reaction product of a 1:1 mole ratio of isophorone diisocyanate and 2-hydroxyethyl acrylate, and 0.4 g of di-n-butyltin dilaurate. The mixture was then stirred at 70°C for about 24 hours to allow reaction. This yielded a compound 2.
Compound 2: (viscosity: 650,000 cps/25°C, weight-average molecular weight (Mw): 8,800, glass transition region: -15°C to 62°C, glass transition temperature: -10°C and 48°C, number of polymerizable groups (acryl groups): 2, weight-average molecular weight per polymerizable group: 4,400)

### [Compound Having Weight-Average Molecular Weight of 2,000 or More Other Than Compound (A)]

UN-2600: urethane acrylate (manufactured by Negami Chemical Industrial Co., Ltd., viscosity: 75,000 to 90,000 cps/25°C, weight-average molecular weight (Mw): 2,500, glass transition region: -10°C to 8°C, glass transition temperature (Tg): -1°C, number of polymerizable groups (acryl groups): 2, weight-average molecular weight per polymerizable group: 1,250)

### [Monomer (B)]

· Monomer (B-1) having an acidic group
   MDP: 10-methacryloyloxydecyl dihydrogen phosphate
· Hydrophobic monomer (B-2) having no acidic group
   D-2.6E: 2,2-bis(4-methacryloyloxypolyethoxyphenyl)propane (average number of moles of ethoxy group added: 2.6)
   3G: triethylene glycol dimethacrylate
   DD: 1,10-decanediol dimethacrylate
   MAEA: N-methacryloyloxyethylacrylamide
· Hydrophilic monomer (B-3) having no acidic group
   HEMA: 2-hydroxyethyl methacrylate

### [Polymerization Initiator (C)]

### · Water-soluble photopolymerization initiator (C-1)

Li-TPO: a lithium salt of phenyl(2,4,6-trimethylbenzoyl)phosphinic acid (a compound represented by the following formula (3)), average particle diameter: 5 µm 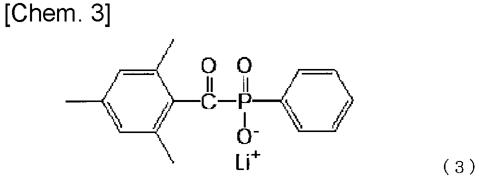
· Water-insoluble photopolymerization initiator (C-2)
   CQ: dl-camphorquinone

### [Filler (D)]

Inorganic filler 1: particulate silica Aerosil^{®} R 972 manufactured by Nippon Aerosil Co., Ltd., average particle diameter: 16 nm, refractive index: 1.46
Inorganic filler 2: silane-treated silica stone powder, refractive index: 1.55
A silica stone powder (quartz, Hi-Silica manufactured by Nitchitsu Co., Ltd. under this trade name) was pulverized with a ball mill to obtain a pulverized silica stone powder. The pulverized silica stone powder had an average particle diameter of 2.2 µm when measured by volume with a laser diffraction particle size distribution analyzer (SALD-2300, manufactured by Shimadzu Corporation). The silane-treated silica stone powder was obtained after the surface treatment of 100 parts by mass of pulverized silica stone powder with 4 parts by mass of γ-methacryloyloxypropyltrimethoxysilane, using an ordinary method.

### [Polymerization Accelerator (E)]

DABE: ethyl 4-(N,N-dimethylamino)benzoate

### [Other]

BHT: 2,6-di-t-butyl-4-methylphenol (a stabilizer, a polymerization inhibitor)

### [Application of Dental Composition to Self-Adhesive Dental Composite Resin or Dental Composite Resin]

### <Examples 1-1 to 1-12 and Comparative Examples 1-1 to 1-7>

The above components were used to prepare pastes. Specifically, the components shown in Tables 1 and 2 were mixed and kneaded at ordinary temperature to prepare pastes (compositions) for the self-adhesive dental composite resins of Examples 1-1 to 1-11, the dental composite resin of Example 1-12, the self-adhesive dental composite resins of Comparative Examples 1-1 to 1-5, and the dental composite resins of Comparative Examples 1-6 and 1-7. These pastes were measured for polymerization shrinkage stress and flexural properties using the methods described below. Tables 1 and 2 show the proportion (parts by mass) of each component of Examples and Comparative Examples, along with the test results.

### [Viscosity Measurement]

The compound (A) having a weight-average molecular weight of 2,000 or more, and UN-2600 were measured for viscosity with a dynamic viscoelasticity meter (rotary rheometerAR 2000, manufactured by TA Instruments) at 25°C, with a plate diameter of 25 mm, a plate gap of 0.50 mm, and a shear rate of 1.0 s⁻¹ (n = 3), and the mean value was calculated.

### [Measurement of Weight-Average Molecular Weight]

Tetrahydrofuran was used as eluent, and a column was prepared by joining two TSKgel SuperMultipore HZM-M columns (manufactured by Tosoh Corporation) and one TSKgel Super HZ 4000 column (manufactured by Tosoh Corporation), end to end. A GPC system HLC-8320 (manufactured by Tosoh Corporation) equipped with a differential refractive index detector (RI detector) was used as GPC device. For measurement, 4 mg of compound (A) having a weight-average molecular weight of 2,000 or more, and 4 mg of UN-2600 were separately dissolved in 5 mL of tetrahydrofuran to prepare a sample solution. With the column oven temperature set to 40°C, 20 µL of the sample solution was injected at an eluent flow rate of 0.35 mL/min, and the resulting chromatogram of the specimen solution was analyzed. Separately, a standard curve relating retention time and molecular weight was created by GPC measurement using ten polystyrene standards having a molecular weight in the 400 to 5,000,000 range. From the chromatograms, the weight-average molecular weight was measured for the compound (A) having a weight-average molecular weight of 2,000 or more, and the compound having a weight-average molecular weight of 2,000 or more, using the standard curve (n = 1).

### [Calculation of Number of Polymerizable Groups]

The number of polymerizable groups was calculated for the compound (A) having a weight-average molecular weight of 2,000 or more, and UN-2600, based on ¹H-NMR measurement results. Measurements were taken for the compound (A) having a weight-average molecular weight of 2,000 or more, and UN-2600 dissolved in deuterated chloroform, using an NMR spectrometer (ULTRA SHIELD 400 PLUS manufactured by Bruker Japan) at room temperature with 16 scans. An estimate of the number of polymerizable groups was calculated from the ratio of an integration value of proton peaks (5.7 to 6.6 ppm) derived from the methacryloyl group and an integration value of proton peaks (4.5 to 5.2 ppm) derived from the urethane bond.

### [Measurement of Glass Transition Temperature and Glass Transition Region]

The compound (A) having a weight-average molecular weight of 2,000 or more, and UN-2600 were separately dissolved in acetone with CQ and DABE to obtain specimens. Specifically, CQ and DABE were dissolved in acetone, 1 mass% each with respect to the compound (A) having a weight-average molecular weight of 2,000 or more, and UN-2600, and the acetone was distilled away to obtain a specimen. The specimen was held between two glass slides, and the glass slides were pressed against the specimen with metal boards until the specimen had a thickness of 500 µm. The specimen was then cured into a cured product by exposing the glass slides to light from both sides, 3 minutes on each side, using a dental LED photoirradiator (alpha-Light V manufactured by J. Morita Corp. under this trade name). The cured product was cut into 2.3 to 2.5 mg with a razor to obtain measurement samples. The measurement sample was then filled into the sample pan of a heat flux DSC measurement device (DSC 214 Polyma manufactured by NETZSCH Japan under this trade name). In the heat flux DSC measurement device, the sample was isothermally heated at -150°C for 5 minutes, and the temperature was increased to 200°C at a rate of temperature increase of 20°C/min in a 40 mL/min nitrogen gas atmosphere to measure glass transition temperature and glass transition region (n = 3). The mean value of measured T_{mg} was determined as the glass transition temperature. The glass transition region was calculated from the mean value of the glass transition temperature T_{ig} on the low-temperature side, and the mean value of the glass transition temperature T_{eg} on the high-temperature side. FIG. 2 shows the measurement result for UN-7600. In the measurement, a baseline shift was detected as Tg.

### [Measurement of Polymerization Shrinkage Stress]

A stainless-steel washer (5.3 mm in inner diameter × 0.8 mm in thickness) after the application of a release agent was installed on a 5.0 mm thick glass plate that had been subjected to sandblasting with a 50 µm alumina powder. The paste of the dental composite resin of each Example and Comparative Example was then filled into the washer. After removing the excess paste, the dental composite resin paste was held between the glass plate and a stainless-steel jig (Ø = 5 mm) that had been separately subjected to sandblasting.

The paste was subjected to 10 seconds of photoirradiation from the glass plate side to cure the dental composition resin, using a dental LED photoirradiator (PenCure 2000 manufactured by J. Morita Corp. under this trade name). The resulting polymerization shrinkage stress was measured with a universal testing machine (Autograph AG-I 100kN, manufactured by Shimadzu Corporation) (n = 3), and the mean value was calculated.

### [Evaluation of Flexural Properties]

The strength was evaluated by conducting a flexure test in compliance with ISO 4049:2009, specifically as follows. The dental composite resin paste was filled into a SUS die (2 mm in length × 25 mm in width × 2 mm in thickness), and glass slides were pressed against the paste (dental composition) from the top and bottom (over a 2 mm × 25 mm surface). The dental composition was cured by applying light through the glass slides from both sides, 5 points on each side, 10 seconds each, using a dental LED photoirradiator (PenCure 2000 manufactured by J. Morita Corp.). The cured product was measured for three-point flexural strength and flexural modulus by conducting a flexural test at a span length of 20 mm and a crosshead speed of 1 mm/min, using a universal testing machine (Autograph AG-I, 100kN, manufactured by Shimadzu Corporation) (n = 5), and the mean value was calculated.

**[Table 1]**

| Components (parts by mass) | | Ex. 1-1 | Ex. 1-2 | Ex. 1-3 | Ex. 1-4 | Ex. 1-5 | Ex. 1-6 | Ex. 1-7 | Ex. 1-8 | Ex. 1-9 | Ex. 1-10 | Ex. 1-11 | Ex. 1-12 |
|---|---|---|---|---|---|---|---|---|---|---|---|---|---|
| Compound (A) having a weight-average molecular weight of 2,000 or more | UN-7600 | 10 | 5 | 15 | 20 | 30 | - | - | - | - | 15 | 5 | 10 |
| | Compound 1 | - | - | - | - | - | 10 | 20 | - | - | - | - | - |
| | Compound 2 | - | - | - | - | - | - | - | 10 | 20 | - | - | - |
| Compound having a weight-average molecular weight of 2,000 or more other than (A) | UN-2600 | - | - | - | - | - | - | - | - | - | - | - | - |
| Monomer (B-1) having an acidic group | MDP | 10 | 10 | 10 | 10 | 5 | 10 | 10 | 10 | 10 | 10 | 10 | - |
| Hydrophobic monomer (B-2) having no acidic group | D-2.6 E | 50 | 55 | 45 | 40 | 40 | 50 | 40 | 50 | 40 | 45 | 55 | 70 |
| | DD | 20 | 20 | 20 | 20 | 20 | 20 | 20 | 20 | 20 | 10 | - | 20 |
| | 3G | - | - | - | - | - | - | - | - | - | 10 | - | - |
| | MAEA | 10 | 10 | 10 | 10 | 5 | 10 | 10 | 10 | 10 | 10 | 10 | - |
| Hydrophilic monomer (B-3) having no acidic group | HEMA | - | - | - | - | - | - | - | - | - | - | 20 | - |
| Polymerization initiator (C) | CQ | 0.2 | 0.2 | 0.2 | 0.2 | 0.2 | 0.2 | 0.2 | 0.2 | 0.2 | 0.2 | 0.2 | 0.3 |
| | Li-TPO | 1 | 1 | 1 | 1 | 1 | 1 | 1 | 1 | 1 | 1 | 1 | - |
| Filler (D) | Inorganic filler 1 | 10 | 10 | 10 | 10 | 10 | 10 | 10 | 10 | 10 | 10 | 10 | 10 |
| | Inorganic filler 2 | 170 | 170 | 170 | 170 | 170 | 170 | 170 | 170 | 170 | 170 | 170 | 170 |
| Polymerization accelerator (E) | DABE | 0.2 | 0.2 | 0.2 | 0.2 | 0.2 | 0.2 | 0.2 | 0.2 | 0.2 | 0.4 | 0.4 | 0.4 |
| Other | BHT | 0.05 | 0.05 | 0.05 | 0.05 | 0.05 | 0.05 | 0.05 | 0.05 | 0.05 | 0.05 | 0.05 | 0.05 |
| Polymerization shrinkage stress | MPa | 7.8 | 8.5 | 7.2 | 6.4 | 5.9 | 8.2 | 6.8 | 8 | 6.7 | 8.3 | 7.8 | 6.6 |
| Flexural modulus | GPa | 6.2 | 6.5 | 6.1 | 5.7 | 5.3 | 6.4 | 6.0 | 6.4 | 6.0 | 5.9 | 4.8 | 4.6 |
| Flexural strength | MPa | 105 | 108 | 100 | 95 | 82 | 102 | 98 | 103 | 96 | 97 | 99 | 100 |

**[Table 2]**

| Components (parts by mass) | | Com. Ex. 1-1 | Com. Ex. 1-2 | Com. Ex. 1-3 | Com. Ex. 1-4 | Com. Ex. 1-5 | Com. Ex. 1-6 | Com. Ex. 1-7 |
|---|---|---|---|---|---|---|---|---|
| Compound (A) having a weight-average molecular weight of 2,000 or more | UN-7600 | - | - | - | - | - | - | - |
| | Compound 1 | - | - | - | - | - | - | - |
| | Compound 2 | - | - | - | - | - | - | - |
| Compound having a weight-average molecular weight of 2,000 or more other than (A) | UN-2600 | - | - | - | 10 | 20 | - | 10 |
| Monomer (B-1) having an acidic group | MDP | 10 | 10 | 10 | 10 | 10 | - | - |
| Hydrophobic monomer (B-2) having no acidic group | D-2.6 E | 60 | 60 | 60 | 50 | 40 | 75 | 70 |
| | DD | 20 | - | - | 20 | 20 | 25 | 20 |
| | 3G | - | 20 | - | - | - | - | - |
| | MAEA | 10 | 10 | 10 | 10 | 10 | - | - |
| Hydrophilic monomer (B-3) having no acidic group | HEMA | - | - | 20 | - | - | - | - |
| Polymerization initiator (C) | CQ | 0.2 | 0.2 | 0.2 | 0.2 | 0.2 | 0.3 | 0.3 |
| | Li-TPO | 1 | 1 | 1 | 1 | 1 | - | - |
| Filler (D) | Inorganic filler 1 | 10 | 10 | 10 | 10 | 10 | 10 | 10 |
| | Inorganic filler 2 | 170 | 170 | 170 | 170 | 170 | 170 | 170 |
| Polymerization accelerator (E) | DABE | 0.2 | 0.2 | 0.2 | 0.2 | 0.2 | 0.4 | 0.4 |
| Other | BHT | 0.05 | 0.05 | 0.05 | 0.05 | 0.05 | 0.05 | 0.05 |
| Polymerization shrinkage stress | MPa | 10.5 | 12.0 | 10.4 | 9.5 | 9.3 | 9.5 | 9.0 |
| Flexural modulus | GPa | 6.6 | 6.8 | 5.3 | 5.3 | 4.6 | 6.2 | 4.6 |
| Flexural strength | MPa | 110 | 121 | 99 | 104 | 110 | 114 | 109 |

As can be seen in Tables 1 and 2, the self-adhesive dental composite resins according to the present invention (Examples 1-1 to 1-11) produced cured products having a flexural strength of 82 MPa or more, a value sufficient for practical applications, and the cured products exhibited a low polymerization shrinkage stress of 8.5 MPa or less. The dental composite resin according to the present invention (Example 1-12) produced a cured product having a flexural strength of 100 MPa, and the cured product exhibited a low polymerization shrinkage stress of 6.6 MPa. In contrast, the polymerization shrinkage stress was 9.3 MPa or more in the self-adhesive dental composite resins (Comparative Examples 1-1 to 1-5) that did not contain a compound (A) having a weight-average molecular weight of 2,000 or more, or that contained a compound having a weight-average molecular weight of 2,000 or more other than (A), as shown in Table 2. The polymerization shrinkage stress was 9.0 MPa or more in the dental composite resins (Comparative Examples 1-6 and 1-7) that did not contain a compound (A) having a weight-average molecular weight of 2,000 or more, or that contained a compound having a weight-average molecular weight of 2,000 or more other than (A). As demonstrated above, the reduction in polymerization shrinkage stress was found to be insufficient in the comparative examples. These results suggest that containing a compound (A) having a weight-average molecular weight of 2,000 or more can achieve relaxation of polymerization shrinkage stress, and can effectively reduce the risk of detachment or marginal leakage in the restorative treatment of relatively deep cavities.

### INDUSTRIAL APPLICABILITY

A dental composition according to the present invention is suited for use as a dental composite resin (particularly preferably, a self-adhesive dental composite resin) or a dental cement in the field of dental treatment.

### Reference Signs List

1: Glass transition temperature 1
2: Glass transition temperature 2
3: Glass transition region

## Claims

1. A dental composition comprising a compound (A) having a weight-average molecular weight of 2,000 or more, a monomer (B), and a polymerization initiator (C), wherein the compound (A) having a weight-average molecular weight of 2,000 or more has a glass transition region including a temperature range of 20 to 40°C, and the monomer (B) excludes the compound (A) having a weight-average molecular weight of 2,000 or more.

2. The dental composition according to claim 1, wherein the compound (A) having a weight-average molecular weight of 2,000 or more has a weight-average molecular weight of less than 50,000.

3. The dental composition according to claim 1 or 2, wherein the compound (A) having a weight-average molecular weight of 2,000 or more has a urethane bond.

4. The dental composition according to claim 3, wherein the compound (A) having a weight-average molecular weight of 2,000 or more is a urethanized (meth)acrylic compound (A-1).

5. The dental composition according to any one of claims 1 to 4, wherein the compound (A) having a weight-average molecular weight of 2,000 or more has a glass transition temperature at 20°C or less.

6. The dental composition according to any one of claims 1 to 5, wherein the compound (A) having a weight-average molecular weight of 2,000 or more is 0.1 to 50 parts by mass in total 100 parts by mass of the compound (A) having a weight-average molecular weight of 2,000 or more, and the monomer (B).

7. The dental composition according to any one of claims 1 to 6, wherein the compound (A) having a weight-average molecular weight of 2,000 or more has two or more glass transition temperatures.

8. The dental composition according to claim 7, wherein the compound (A) having a weight-average molecular weight of 2,000 or more has one or more glass transition temperatures in a temperature region of -100°C or more and 20°C or less, and one or more glass transition temperatures in a temperature region of 20°C or more and less than 80°C.

9. The dental composition according to any one of claims 1 to 8, wherein the monomer (B) comprises a monomer (B-1) having an acidic group.

10. The dental composition according to any one of claims 1 to 9, which further comprises a filler (D).

11. The dental composition according to any one of claims 1 to 10, wherein the compound (A) having a weight-average molecular weight of 2,000 or more has a polymerizable group, and has a weight-average molecular weight of 1,250 or more and less than 20,000 per its polymerizable group.

12. A dental composite resin comprising a dental composition of any one of claims 1 to 11.

13. A self-adhesive dental composite resin comprising a dental composition of any one of claims 1 to 11.

14. A dental cement comprising a dental composition of any one of claims 1 to 11.
